# EUROPEAN PATENT APPLICATION

(11) **EP 3 815 699 A1**
(43) Date of publication of application: **05.05.2021**
(21) Application number: 19205530.9
(22) Date of filing: 28.10.2019
(51) Int. Cl.: A61K 33/36, A61K 45/06, A61P 31/20, G01N 33/569

(54) **NEW MEANS AND METHODS FOR THERAPY AND DIAGNOSIS OF ADENOVIRAL INFECTION**

(71) Applicant: Technische Universität München, 80333 München (DE)
(72) Inventor: SCHREINER, Sabrina, 94560 Offenberg (DE); HOFMANN, Samuel, 85356 Freising (DE)
(74) Representative: Grünecker Patent- und Rechtsanwälte PartG mbB

(57) **Abstract**

The present invention relates to the field of adenovirus infection, in particular to therapy and diagnosis of an adenovirus infection in a subject. Specifically, the present invention relates to the use of arsenic trioxide (As₂O₃) for treating or preventing an adenovirus infection in a subject. Furthermore, the present invention relates to a method of detecting an adenovirus infection based on detecting SUMOylation of adenoviral E2A protein in a patient sample.

## Description

### FIELD OF THE INVENTION

The present invention relates to the field of adenovirus infection, in particular to therapy and diagnosis of an adenovirus infection in a subject. Specifically, the present invention relates to the use of arsenic trioxide (As₂O₃) for treating or preventing an adenovirus infection in a subject. Furthermore, the present invention relates to a method of detecting an adenovirus infection based on detecting SUMOylation of adenoviral E2A protein in a patient sample.

### BACKGROUND OF THE INVENTION

Adenovirus infections are widely distributed in human populations. Human adenoviruses (HAdV) are the causative agent of acute and chronic infections, associated with a variety of clinical symptoms such as gastroenteritis, keratoconjunctivitis, pneumonia, hepatitis and encephalitis. In the absence of protective immunity, HAdV infections can become lethal. Alarmingly, various outbreaks of highly pathogenic, pneumotropic HAdV types were reported, causing severe and lethal respiratory diseases in immunocompetent patients. Adenovirus is known to be a persistent virus, remaining latent in previously infected individuals, and it can be reactivated by various factors, leading to the development of more severe diseases. In immunocompromised populations, like organ transplant or stem cell transplant recipients and patients suffering from cancer or additional chronic infections, HAdV infections can lead to severe complications with high morbidity and mortality (Razonable, R.R. and A.J. Eid, Viral infections in transplant recipients. Minerva Med, 2009. 100(6): p. 479-501).

HAdV are large, non-enveloped icosahedral viruses containing a double stranded (ds) DNA genome. HAdV is reliant on the host cell for replication and virus growth. In particular, HAdV forms replication and transcriptional domains called replication centers (RCs) that are marked by viral protein E2A. This early HAdV DNA binding protein is involved in viral replication, regulation of viral gene expression, mRNA stability and virion assembly. Formation of RCs often takes place in close proximity to promyelocytic leukemia nuclear bodies (PML-NBs) in the nucleus of the host cell, suggesting a role of some PML components in viral replication. PML-NBs are interferon inducible, matrix-associated multiprotein complexes, and various proteins localize in these aggregate structures, including transient or constitutive components such as PML, Daxx (death domain-associated protein), SUMO, or Sp100. PML-NBs exhibit antiviral activity by impairing efficient viral replication. To counteract these measurements, several DNA viruses express early viral proteins, which interact with PML-NBs and specifically interfere with the antiviral function.

SUMO is a small ubiquitin-like modifier that regulates PML-NB function, integrity and formation by posttranslational modification (PTM) of PML and PML-associated proteins. SUMO proteins are covalently conjugated to substrate proteins via a three step enzymatic pathway and usually attach to lysine residues of the substrates, which are part of the short SUMO conjugation motif (SCM) consensus sequence ΨKXE (Ψ is a large hydrophobic amino acid, generally isoleucine, leucine, or valine; K is the lysine residue that is modified; X is any amino acid residue; and E is a glutamic acid). This process is called SUMOylation, a post-translational modification involved in various cellular processes.

Despite HAdV being subject to extensive research and having high clinical significance, neither a specific anti-adenoviral treatment nor an efficient vaccination strategy are available yet (Martinez-Aguado, P., et al., Anti-adenovirus drug discovery: potential targets and evaluation methodologies. Drug Discov Today, 2015. 20(10): p. 1235-42). Hence, there is still a need for effective treatment/prevention strategies that are useful to target adenovirus infections.

The present invention addresses these needs based on the surprising finding that arsenic trioxide (As₂O₃/ATO) treatment represents a general antiviral strategy against DNA viruses.

Arsenic trioxide (As₂O₃/ATO) is a compound originally used since over 2000 years in traditional Chinese medicine. In 1992, ATO was first described to induce an efficient and complete response in patients suffering from acute promyelocytic leukemia (APL), prior to approval by the U.S. Food and Drug Administration (FDA) for APL treatment in 2000 (Soignet, S.L., et al., United States multicenter study of arsenic trioxide in relapsed acute promyelocytic leukemia. J Clin Oncol, 2001. 19(18): p. 3852-60).

### SUMMARY OF THE INVENTION

The present invention is based on the surprising finding that arsenic trioxide (As₂O₃/ATO) treatment represents a general antiviral strategy against DNA viruses. The new means and methods for therapy and diagnosis of adenoviral infection provided by the present invention are based, to a large extent, on the surprising finding that ATO acts as a potent inhibitor of adenovirus infection, in particular human adenovirus (HAdV) infection, due to inhibition of adenoviral replication by de-SUMOylation of early adenoviral protein E2A. Thus, ATO treatment negatively regulates an efficient adenovirus, in particular HAdV, infection cycle.

Accordingly, the present invention provides:
[1] Arsenic trioxide (As₂O₃) for use in the treatment or prevention of an adenovirus infection in a subject.
[2] Arsenic trioxide for the use according to [1], wherein arsenic trioxide is preventing and/or inhibiting recurrent, persistent, and/or de novo viral infection.
[3] Arsenic trioxide for the use according to [1] or [2], wherein arsenic trioxide is preventing and/or inhibiting replication of adenovirus.
[4] Arsenic trioxide for the use according to [1], wherein the arsenic trioxide is reducing the adenoviral load.
[5] Arsenic trioxide for the use according to any one of [1] to [4], wherein the subject is a subject suffering from, or at risk of contracting, a disease or disorder associated with, or caused by, an adenovirus infection.
[6] Arsenic trioxide for the use according to [5], which is for lessening the severity of clinical symptoms in the subject suffering from the disease or disorder associated with, or caused by, an adenovirus infection.
[7] Arsenic trioxide for the use according to [5] or [6], wherein the disease or disorder associated with, or caused by, an adenovirus infection is any one of a disease or disorder of the respiratory system, conjunctivitis, preferably keratoconjunctivitis, gastroenteritis, pneumonia, hepatitis, encephalitis, or cystitis.
[8] Arsenic trioxide for the use according to any one of [1] to [7], wherein the subject is an immunocompromised subject, preferably wherein the immunocompromised subject is a solid-organ transplant recipient, a stem cell transplant recipient, or a subject suffering from cancer and/or an additional chronic infection.
[9] Arsenic trioxide for the use according to any one of [1] to [8], further comprising treatment of the subject with an anti-adenovirus agent, which is not arsenic trioxide.
[10] Arsenic trioxide for the use according to [9], wherein the anti-adenovirus agent is to be administered to the subject before, after, or simultaneously with arsenic trioxide.
[11] Arsenic trioxide for the use according to any one of [1] to [10], wherein the arsenic trioxide is to be administered at a low dose of 0.15 - 0.16 mg/kg/d or less.
[12] A kit comprising a therapeutically effective amount of arsenic trioxide (As₂O₃) and instructions for the administration of arsenic trioxide to a subject suffering from an adenovirus infection, or at risk of being infected with an adenovirus.
[13] The kit of [12], further comprising an anti-adenovirus agent, which is not arsenic trioxide.
[14] A method of detecting an adenovirus infection in a subject, comprising detecting SUMOylation of adenovirus E2A protein, preferably detecting E2A SUMO conjugates, in a sample obtained from the subject.
[15] Use of SUMOylated adenovirus E2A protein, or E2A SUMO conjugates, as a marker for detecting an adenovirus infection in a subject.
[16] Use of SUMOylated adenovirus E2A protein, or E2A SUMO conjugates, in a method for detecting an adenovirus infection in a subject.
[17] A diagnostic assay or kit for detecting an adenovirus infection in a subject, comprising means for detecting SUMOylation of adenovirus E2A protein, preferably means for detecting E2A SUMO conjugates, in a sample obtained from the subject.
[18] Arsenic trioxide for the use according to any one of [1] to [11], or the kit according to [12] or [13], or the method of [14], or the use according to [15] or [16], or the diagnostic assay or kit according to [17], wherein the subject is a mammal, preferably a human.
[19] Arsenic trioxide for the use according to any one of claims 1 to 11 and 18, or the kit according to any one of [12], [13], and [18], or the method of [14] or [18], or the diagnostic assay or kit according to [17] or [18], or the use according to any one of [15], [16] and [18], wherein the adenovirus is human adenovirus (HAdV).
[20] Arsenic trioxide for the use according to any one of [1] to [11], [18] and [19], or the kit according to any one of [12], [13], [18], and [19], wherein the arsenic trioxide is formulated for oral administration, administration via inhalation, topical administration, or intravenous administration.

### DESCRIPTION OF THE DRAWINGS

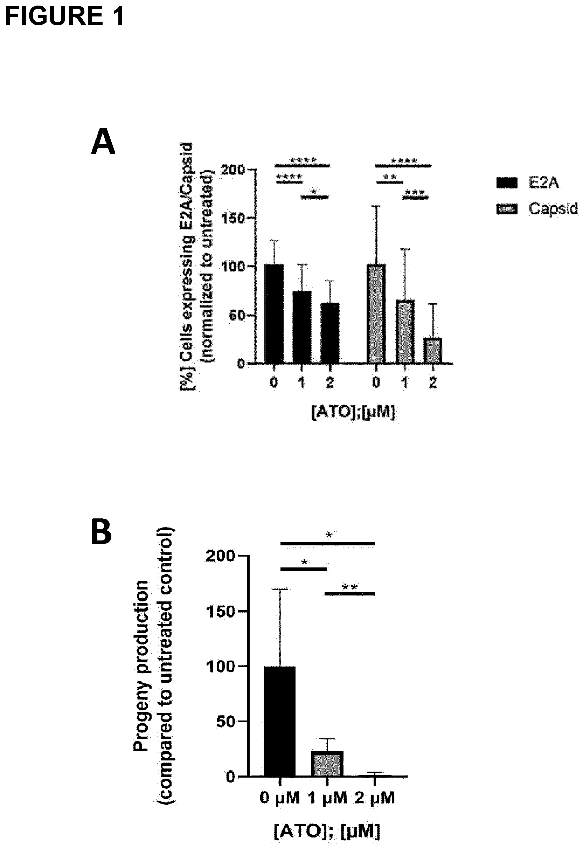

**Figure 1****: ATO efficiently reduces HAdV infectivity** (cf. Example 1). H1299 cells were infected with HAdV-C5 wt at a multiplicity of 5 FFU/cell, treated with the depicted concentrations of ATO at 2 h.p.i., fixed 48 h.p.i. with 4 % PFA and double labeled with mAb B6-8 (α-E2A) and pAb L133 (α-Capsid). Primary antibodies were detected using Alexa488 (E2A, green) and Alexa568 (Capsid, red) conjugated secondary antibodies. **(A)** The number of E2A and Capsid expressing cells, respectively, was counted in n=40 overview pictures (not shown), normalized to untreated, infected cells, and represented in bar charts. Bar charts represent average values and standard deviations based on four independent experiments. **(B)** H1299 cells were infected with HAdV-C5 at a multiplicity of 20 FFU/cell, and treated with the depicted concentrations of ATO at 2 h.p.i.. Viral particles were harvested 48 h.p.i. and virus yield was determined by quantitative E2A immunofluorescence staining in 293 cells. Bar charts represent average values and standard deviations based on three independent experiments. Statistically significant differences were determined using a two-sided Welch's t-test. *: P ≤ 0.05, **: P ≤ 0.01, ***: P ≤ 0.001, ****: P ≤ 0.0001.

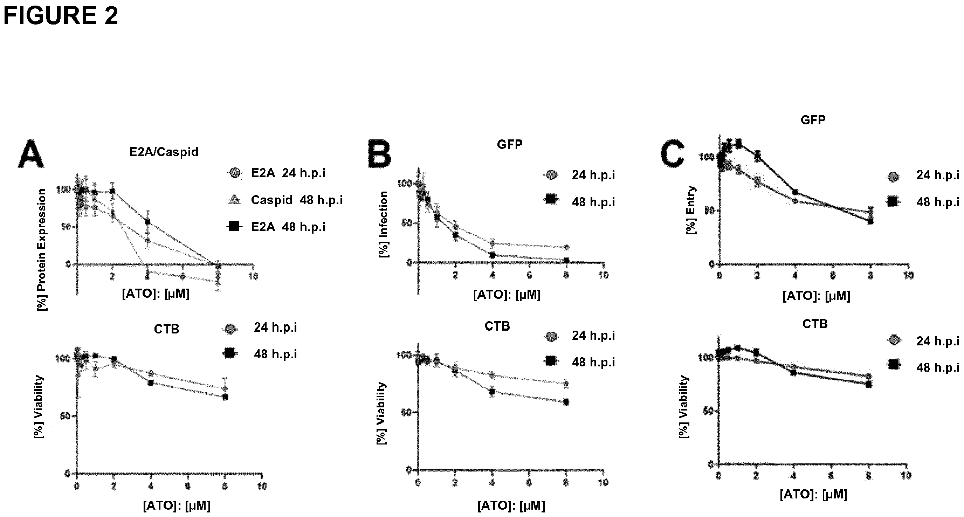

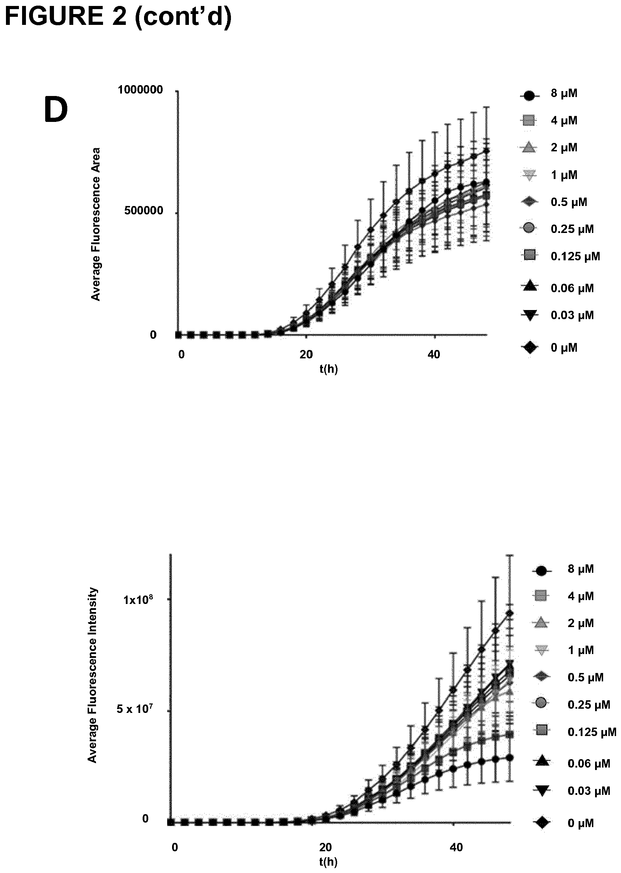

**Figure 2****: ATO induces a dose dependent reduction of HAdV infectivity with minor effects on cell viability** (cf. Example 2). **(A)** H1299 cells were infected with HAdV-C5 wt at a multiplicity of 20 FFU/cell and treated with the depicted concentrations of ATO at 2 h.p.i.. 24 or 48 h.p.i., cell viability was assessed using the Promega CellTiter-Blue Cell Viability Assay system, prior to fixation with 4 % PFA and cells were double labeled with mAb B6-8 (α-E2A) and pAb L133 (α-Capsid). Primary antibodies were detected using Alexa488 (E2A, green) and Alexa647 (Capsid, red) conjugated secondary antibodies. Fluorescence intensity was measured using a Tecan Infinite 200M plate reader using an excitation and emission wavelength of 488 and 520nm for Alexa488 and 640 and 670nm for Alexa647 respectively. Fluorescence intensity was normalized to untreated, infected cells at 48h p.i.. xy charts represent average values and standard deviations based on three independent experiments measured in triplicates. **(B)** H1299 cells were infected with an HAdV-C5 delta E3 virus, encoding a CMV promoter driven eGFP expression cassette, at a multiplicity of 20FFU/cell and treated with the depicted concentrations of ATO at 2h p.i.; 24 or 48 h p.i. cell viability was assessed using the Promega CellTiter-Blue Cell Viability Assay system, and GFP fluorescence intensity was measured using a Tecan Infinite 200M plate reader using an excitation and emission wavelength of 488 and 520nm. Fluorescence intensity was normalized to untreated, infected cells at 48h p.i.; xy charts represent average values and standard deviations based on three independent experiments measured in triplicates. **(C)** H1299 cells were infected with an eGFP expressing HAdV-C5 based first generation adenoviral vector at a multiplicity of 20 FFU/cell and treated with the depicted concentrations of ATO at 2h p.i.; 24 or 48h p.i., cell viability was assessed using the Promega CellTiter-Blue Cell Viability Assay system, and GFP fluorescence intensity was measured using a Tecan Infinite 200M plate reader using an excitation and emission wavelength of 488 and 520nm. Fluorescence intensity was normalized to untreated, infected cells at 48h p..i.; xy charts represent average values and standard deviations based on three independent experiments measured in triplicates. **(D)** A549 cells were infected with an HAdV-C5 delta E3 virus, encoding a CMV promoter driven eGFP expression cassette, at a multiplicity of 20FFU/cell and treated with the depicted concentrations of ATO at 2h p.i.. GFP-fluorescence, as well as cell growth via phase contrast-imaging was assessed for 48 h with a 2h increment using an IncuCyte S3 Live-Cell Analysis System. Average Fluorescence Area (top) and Average Fluorescence intensity (bottom) were determined and plotted over the time.

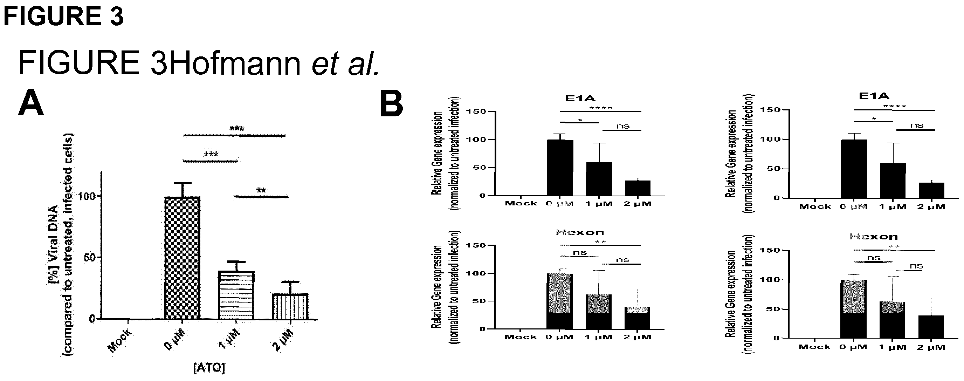

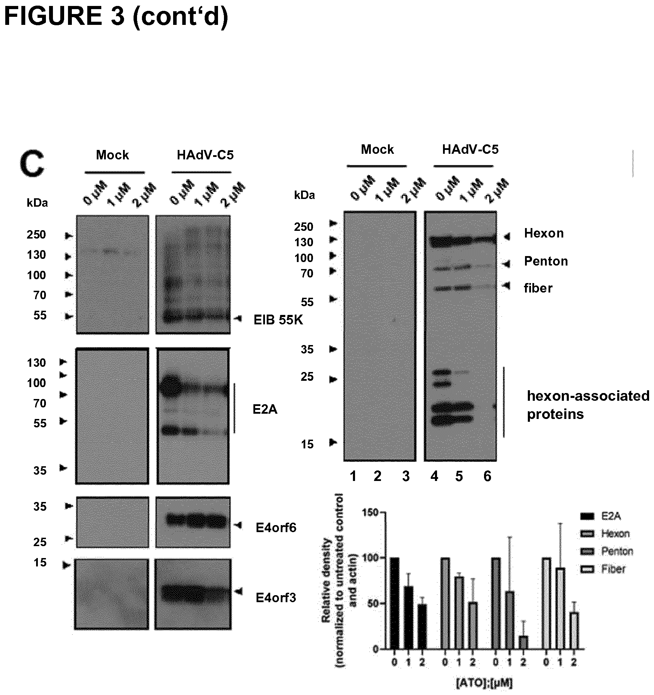

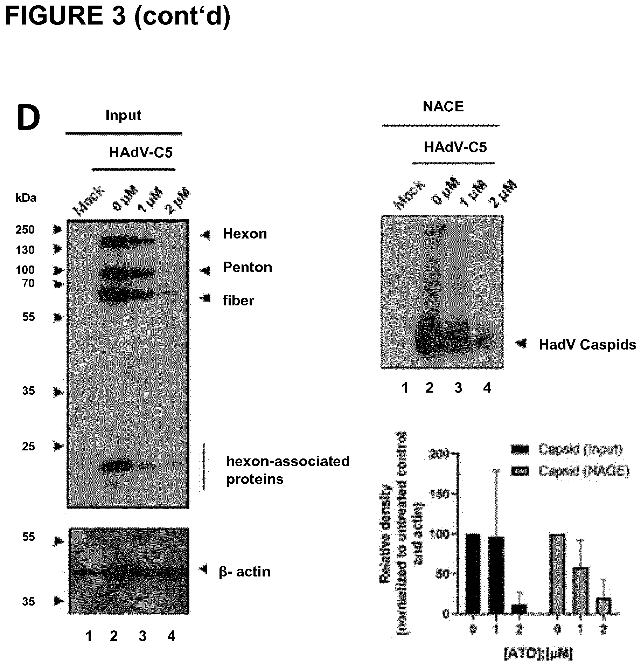

**Figure 3****: ATO interferes with HAdV gene expression and capsid formation** (cf. Examples 3, 4 and 5). H1299 cells were infected with HAdV-C5 wt at a multiplicity of 20FFU/cell and treated with the depicted concentrations of ATO at 2 h.p.i.. **(A)** Cells were harvested 24h p.i., total DNA was isolated and subjected to qPCR using primers specific for the viral e1b coding region. As internal control, primers for GAPDH were used. Bar charts represent average values and standard deviations based on two independent experiments measured in triplicates. **(B)** Cells were harvested 48h p.i. and total mRNA was isolated using TRIzol, reverse transcribed and quantified by RT-PCR using primers specific for HAdV E1A and hexon. The data was normalized to the respective 18S mRNA levels. Bar charts represent average values and standard deviations based on two independent experiments measured in triplicates. **(C)** 48 h.p.i., proteins from total-cell protein lysates were separated by SDS-PAGE and subjected to immunoblotting using mAb 2A-6 (α-E1B-55K), mAb RSA3 (α-E4orf6), mAb B6-8 (α-E2A), mAb6A11 (α-E4orf3), pAb L133 (a-Capsid), pAb NB100-59787 (a-PML), and mAb AC-15 (α-β-actin). Relevant proteins are depicted on the right, molecular weights in kDa on the left of each blot, respectively. For quantification of protein expression, densitometric analysis of detected bands was performed using ImageJ (version 1.45s). Relative protein expression was normalized on the respective α-β-actin steady state levels. Bar charts represent average values and standard deviations based on three independent experiments. **(D)** Cells were lysed using a low stringent NP-40 lysis buffer. Native lysates were separated by agarose gel electrophoresis and further analyzed by immunoblotting using pAb L133 (α-Capsid). For determination of protein steady state levels, lysates were denatured using Laemmli buffer, separated by SDS-PAGE and subjected to immunoblotting using pAb L133 (a-Capsid) and mAb AC-15 (α-β-actin). Relevant proteins are depicted on the right, molecular weights in kDa on the left of each blot, respectively. For quantification of protein expression, densitometric analysis of detected bands was performed using ImageJ (version 1.45s). Relative protein expression was normalized on the respective α-β-actin steady state levels. Relative Capsid levels as detected by NAGE were normalized to input Capsid expression levels, as well as the respective α-β-actin steady state levels. Bar charts represent average values and standard deviations based on three independent experiments. Statistically significant differences were determined using a two-sided Welch's t-test. *: P ≤ 0.05, **: P ≤ 0.01, ***: P ≤ 0.001, ****: P ≤ 0.0001.

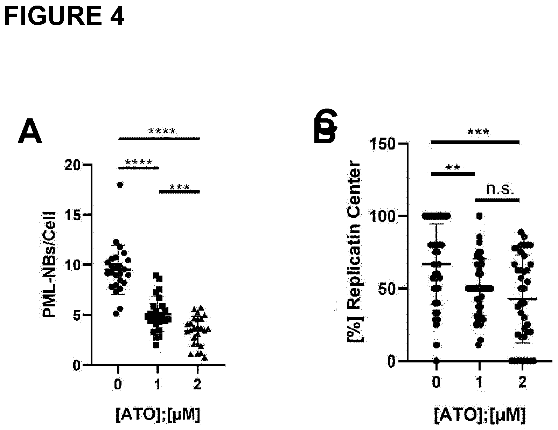

**Figure 4****: ATO interferes with efficient HAdV replication center formation and number of PML-NBs** (cf. Example 6). H1299 cells were infected with HAdV-C5 wt at a multiplicity of 20 FFU/cell, treated with the depicted concentrations of ATO at 2h p.i., fixed 48 h.p.i. with 4 % PFA and double labeled with mAb B6-8 (α-E2A) and pAb NB100-59787 (α-PML). Primary antibodies were detected using Alexa488 (PML, green) and Alexa647 (E2A, red) conjugated secondary antibodies. **(A)** Number of PML-NBs per cell in uninfected cells was determined using Volocity for at least n=461 cells from two independent biological replicates. Statistically significant differences were determined using a two-sided Welch's t-test. *: P ≤ 0.05, **: P ≤ 0.01, ***: P ≤ 0.001, ****: P ≤ 0.0001. **(B)** The proportion of infected cells showing formation of HAdV replication centers marked by the viral protein E2A was determined by counting for at least n=212 cells and normalization to untreated infected cells.

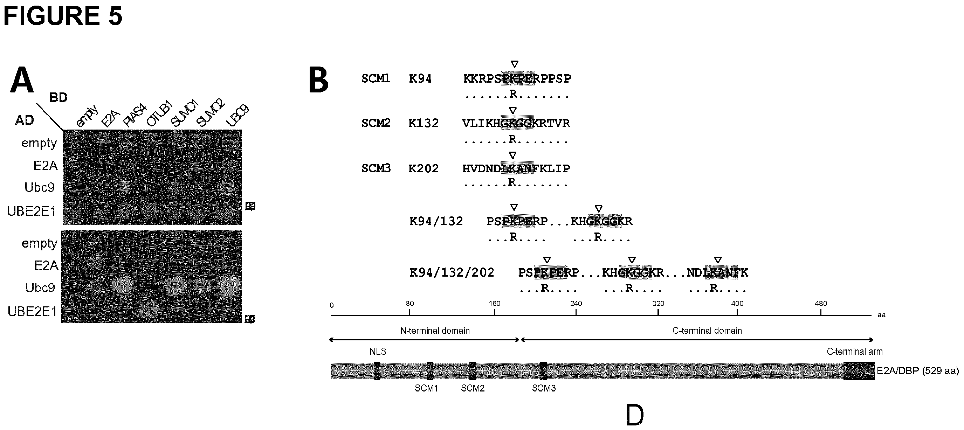

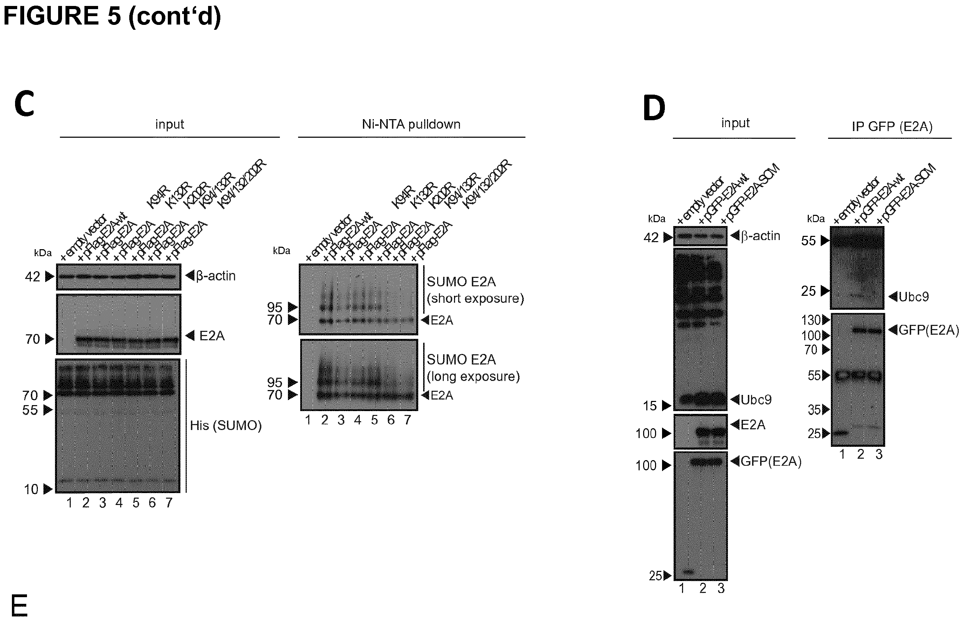

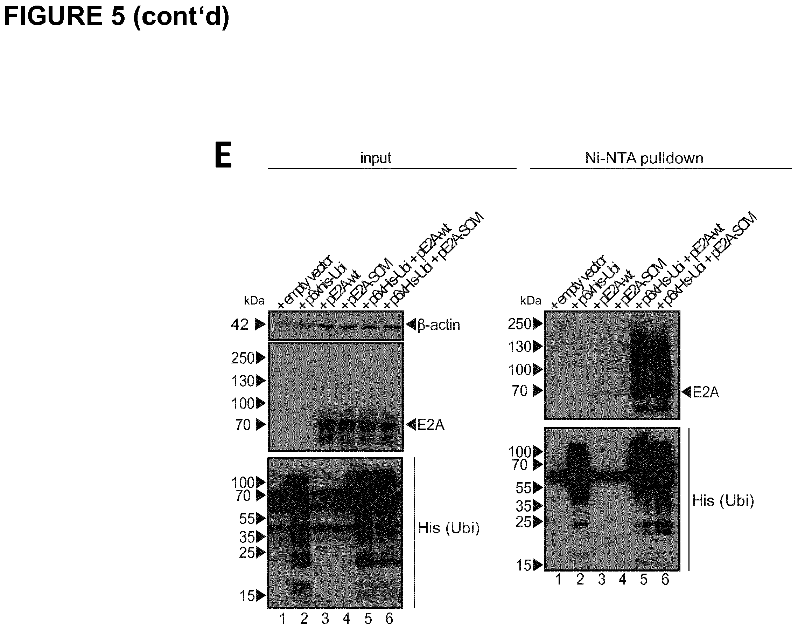

**Figure 5****: E2A harbors at least three SUMO conjugation motifs** (cf. Example 7). **(A)** E2A, PIAS4, 0TUB1, SUM01, SUM02, Ubc9, UBE2E1 and empty vector constructs were co-transformed into the yeast strain PJ69- 7A as indicated. AD and BD empty vectors were used as controls (empty). Co-transformation was confirmed by plating cells on _LEU-TRP agar plates (+HIS). Interaction of candidate proteins was assessed by spotting cells on -LEU-TRP-HIS-agar plates. **(B)** Schematic representation of the E2A protein structure with amino acid (aa) numbering above. Known protein domains are indicated by NLS (nuclear localization signal), N-terminal arm, and C-terminal arm. Below are depicted point mutations that change lysine residue into arginine within the SUMO conjugation motifs (SCM). **(C)** HeLa cells stably expressing 6His-SUMO-2 were transfected with 5 pg of pCMX3b-Flag E2A (pFlag-E2A-wt) (lane 2) and pCMX3b-Flag E2A (pFlag-E2A) variants containing mutations in SCMs (lanes 3-7). 48 h after transfection whole-cell lysates were prepared with guanidinium chloride buffer, subjected to Ni- NTA purification of 6His-SUM0 conjugates and separated by SDS-PAGE prior to immunoblot analyses. Ni-NTA purified proteins and input levels of cell lysates were detected using mAb B6-8 (a- E2A), mAb 6His (anti-6xHis-tag) and mAb AC- 15 (anti-β-actin). Molecular weights in kDa are indicated on the left, relevant proteins on the right. **(D)** H1299 cells were transfected with 5 pg of pGFP-E2A-wt (lane 2) and pGFP-E2A-SCM (lane 3). After 30 h, cells were harvested, and total-cell lysates were prepared. Immunoprecipitation was performed using pAb ab290 (anti-GFP) and proteins were subjected to immunoblot analyses. Input levels of total-cell lysates and co-precipitated proteins were detected using mAb B6-8 (anti-E2A), pAb ab290 (anti-GFP), mAb C-12 (anti-Ubc9) and mAb AC-15 (anti-β-actin). Molecular weights in kDa are indicated on the left, relevant proteins on the right. **(E)** H1299 cells were transfected with 10pg of p6xHis-Ubi and 5pg of pE2A-wt or pE2A-SCM. 30 h after transfection cells were harvested and whole-cell lysates were prepared with guanidinium chloride buffer. After Ni-NTA purification of 6His-SUMO conjugates and separation by SDS-PAGE immunoblot analyses was performed using mAb B6-8 (anti-E2A), mAb 6His (a-6xHis-tag) and mAb AC-15 (anti-β-actin). Molecular weights in kDa are indicated on the left, relevant proteins on the right.

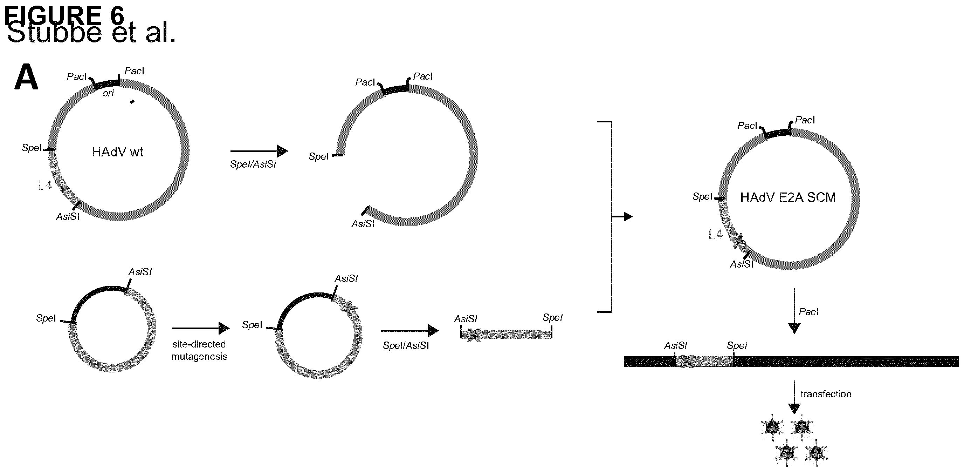

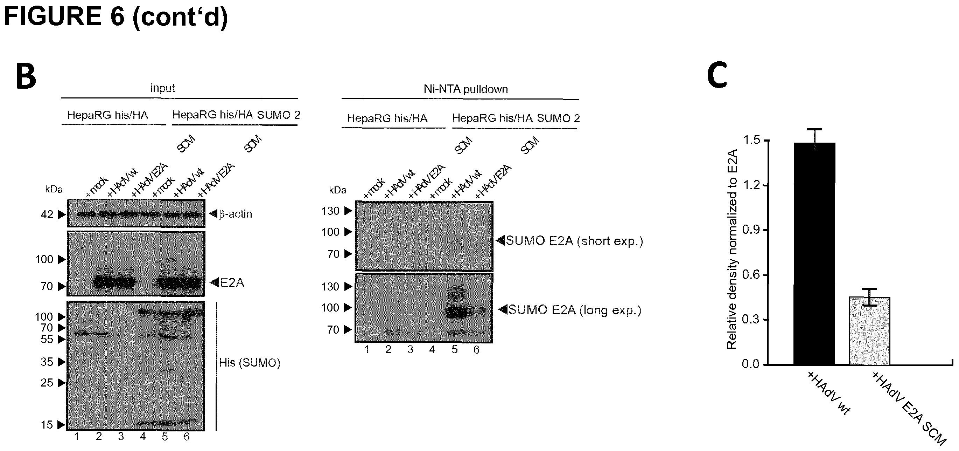

**Figure 6****: Generation and validation of the E2A SCM mutant virus** (cf. Example 8). **(A)** Point mutations were inserted into the E2A gene in the late HAdV region L4 from nt 21438-27081 (pL41513) by site-directed mutagenesis. The L4 plasmid and bacmid pH5pg4100 were linearized by AsiSI plus Spel digestion and the 5.6 kb AsiSI/Spel fragment from pH5pg4100 was replaced with the corresponding fragment from the mutated pL4 construct. The viral genome was released by Pacl digestion and used to transfect H1299 cells followed by virus propagation and isolation. **(B)** HepaRG cells stably expressing His/HA or His/HA-SUMO-2 were infected with wt HAdV and HAdV E2A SCM mutant at a multiplicity of 20 FFU/cell. The cells were harvested 24 h p.i., whole-cell lysates were prepared with guanidinium chloride buffer, subjected to Ni-NTA purification of 6His-SUMO conjugates and separated by SDS-PAGE. Input levels of total-cell lysates and Ni-NTA purified conjugates were detected with mAb B6-8 (anti-E2A), mAb 6His (a-6xHis-tag) and mAb AC- 15 (anti-β-actin). Molecular weights in kDa are indicated on the left, relevant proteins on the right. **(C)** Densitometry analysis of E2A SUMOylation levels in panel B (lanes 5 and 6), quantified with ImageJ (version 1.45s) software, normalized to the respective steady state E2A levels in HAdV wt and HAdV E2A SCM infected cells. Bar plot represents average values and standard deviations calculated based on three separate experiments.

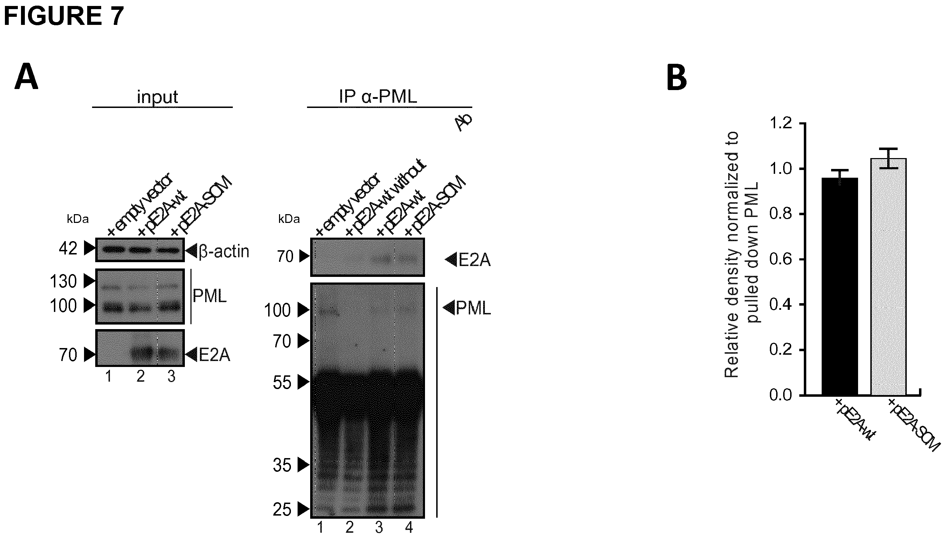

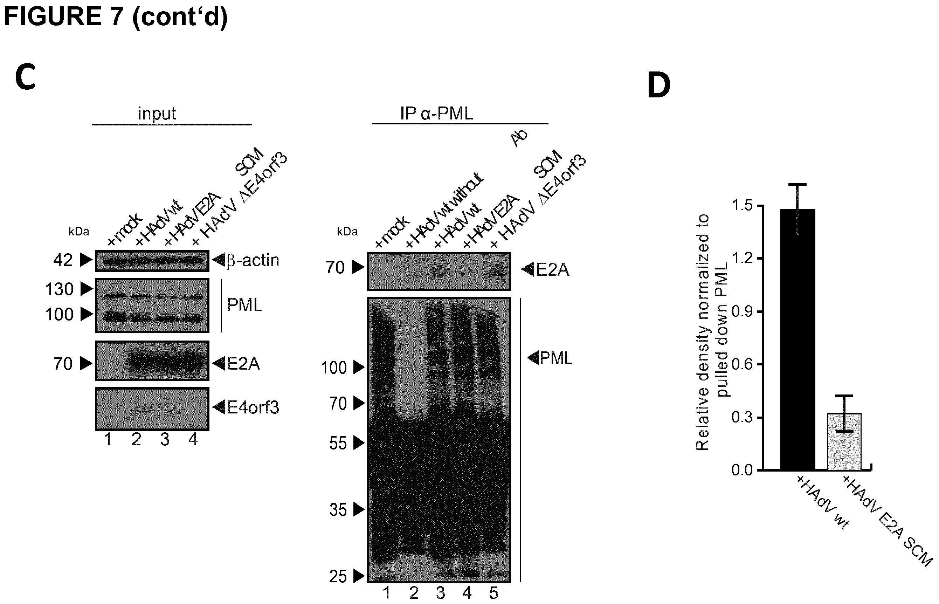

**Figure 7****: E2A SUMOylation controls E2A/PML and PML/PML interactions** (cf. Example 9). **(A)** HepaRG cells transfected with 5 pg of pE2A-wt (lane 2) and pE2A-SCM (lane 3) were harvested 30 h after transfection. After preparation of total cell lysates immunoprecipitation of PML was performed using polyclonal rabbit antibody raised against PML protein pAb NB100-59787 (a-PML) and proteins resolved by SDS-PAGE were visualized by immunoblotting. Input levels of total-cell lysates and co-precipitated proteins were detected using mAb B6-8 (a-E2A), pAb NB100-59787 (a-PML) and mAb AC-15 (α-β-actin). Molecular weights in kDa are indicated on the left, relevant proteins on the right. **(B)** Densitometry analyses of E2A levels immunoprecipitated with PML in panel A (lanes 3 and 4) were quantified with ImageJ (version 1.45s) software, normalized to respective levels of immunoprecipitated PML in pE2A-wt and pE2A-SCM transfected cells. Bar plot represents average values and standard deviations calculated based on data obtained in three separate experiments. **(C)** HepaRG cells were infected with HAdV wt (left panel lane 2, right panel lane 3), HAdV E2A SCM mutant (left panel lane 3, right panel lane 4) and HAdV E4orf3 mutant (left panel lane 4, right panel lane 5) at a multiplicity of 20 FFU/cell, before harvesting cells 24 h after infection and preparing total cell lysates. After immunoprecipitation by PML using polyclonal rabbit Ab raised against PML protein NB100-59787 (anti-PML), proteins were subjected to immunoblot analyses. Input levels of total-cell lysates and co-precipitated proteins were detected using mAb B6-8 (anti-E2A), pAb NB100-59787 (anti-PML), mAb AC-15 (anti-β-actin) and E4orf3 (6A11). Molecular weights in kDa are indicated on the left, relevant proteins on the right. **(D)** Densitometry analyses of E2A coimmunoprecipitated with PML in panel C (right panel lanes 3 and 4), quantified with ImageJ (version 1.45s) software, normalized to respective levels of immunoprecipitated PML in HAdV wt and HAdV E2A SCM infected cells. Average values and standard deviations represented on bar plot were calculated based on three independent experiments.

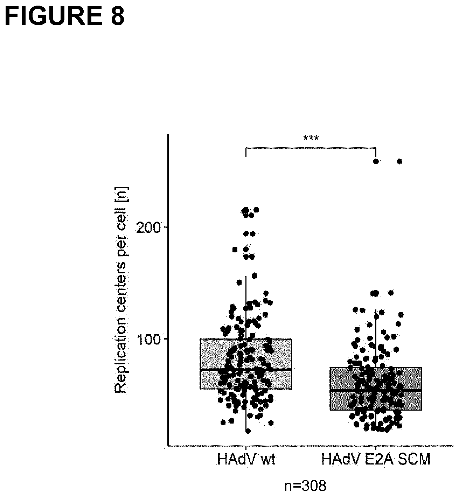

**Figure 8****: Abundance of HAdV RC depends on E2A SUMOylation** (cf. Example 10). Using immunofluorescence microscopy RCs were counted per cell during infection with HAdV wt and HAdV E2A SCM using Volocity software. Numbers of replication centers were plotted in box plots. P values were calculated employing a two-sided Mann-Whitey test.

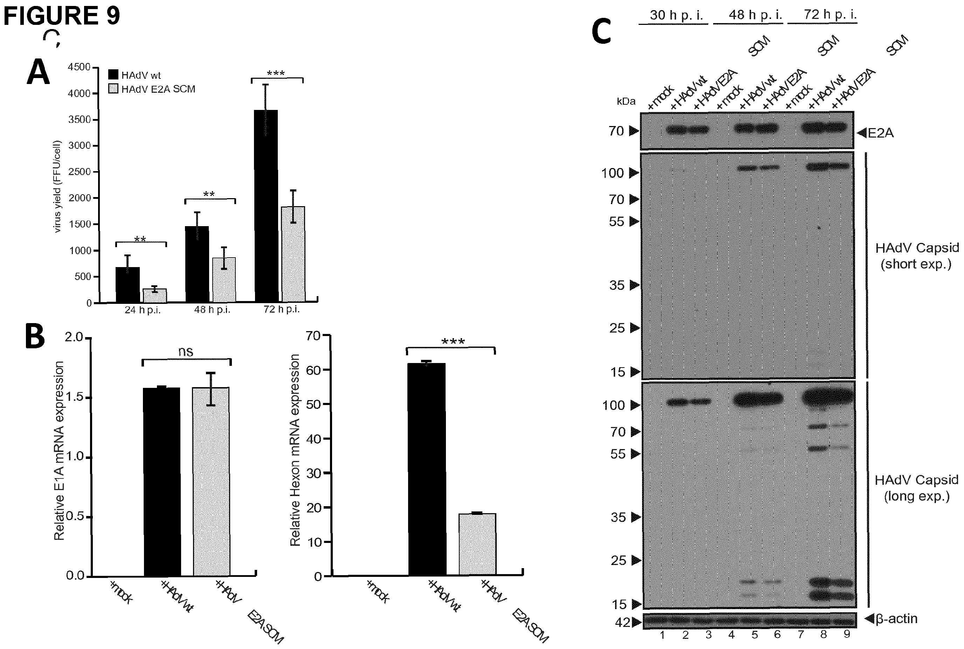

**Figure 9****: E2A SUMOylation promotes HAdV replication** (cf. Example 11). **(A)** Viral particles were harvested 24, 48 and 72 h p.i. and virus yield was determined by quantitative capsid immunofluorescence staining in 2E2 cells. Statistically significant differences were assessed using a two-sided Mann-Whitney test. **(B)** Cells were harvested 30 h p.i. and total RNA was extracted, reverse transcribed and quantified by RT-PCR using primers specific for HAdV E1A (left plot) and Hexon (right plot). The data was normalized to respective GAPDH mRNA levels. Statistics were calculated using a student's t test. **(C)** Cells were harvested 30, 48 and 72 h p.i. Proteins from total-cell extracts were separated by SDS-PAGE and subjected to immunoblotting using mAb AC-15 (anti-β-actin), and B6-8 (anti-E2A). Anti HAdV-C5 rabbit polyclonal serum LI 33 was used for detection of capsid proteins. Molecular weights in kDa are indicated on the left, relevant proteins on the right.

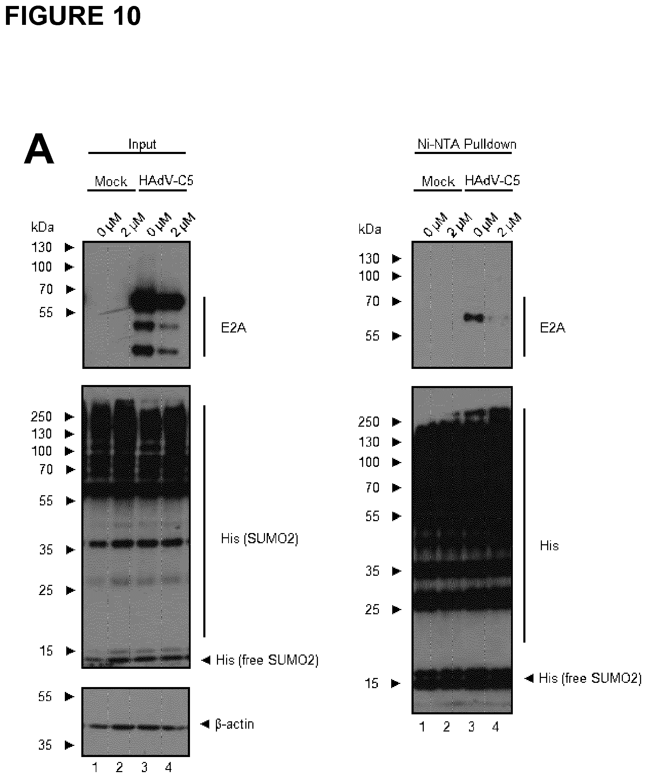

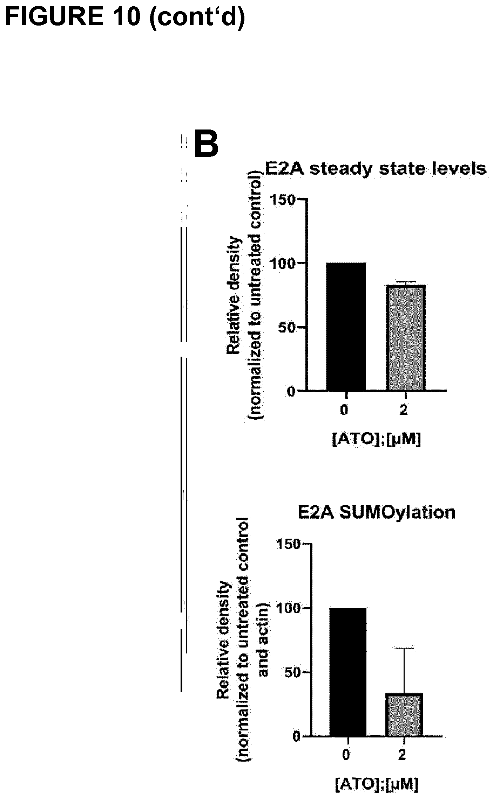

**Figure 10****: ATO highly reduces SUMO2 modification of E2A** (cf. Example 12). **(A)** H1299 cells were transfected with 10µg of p6xHis-SUMO2 for 4h. After transfection, cells were infected with HAdV-C5 wt at a multiplicity of 20 FFU/cell, treated with 2µM of ATO and harvested 24h p.i.. Whole-cell lysates were prepared with guanidinium chloride buffer and subjected to Ni-NTA purification of 6His-SUMO2 conjugates. After Ni-NTA purification, the 6His-SUMO2 conjugates, as well as proteins from total-cell protein lysates were separated by SDS-PAGE and subjected to immunoblotting using mAb B6-8 (α-E2A), mAb 6His (α-6xHis-tag) and mAb AC-15 (α-β-actin). Relevant proteins are depicted on the right, molecular weights in kDa on the left of each blot, respectively. **(B)** For quantification of protein expression, densitometric analysis of detected bands was performed using ImageJ (version 1.45s). Relative protein expression was normalized on the respective α-β-actin steady state levels. The degree of E2A SUMO2 modification was further normalized on E2A steady state levels. Bar charts represent average values and standard deviations based on two independent experiments.

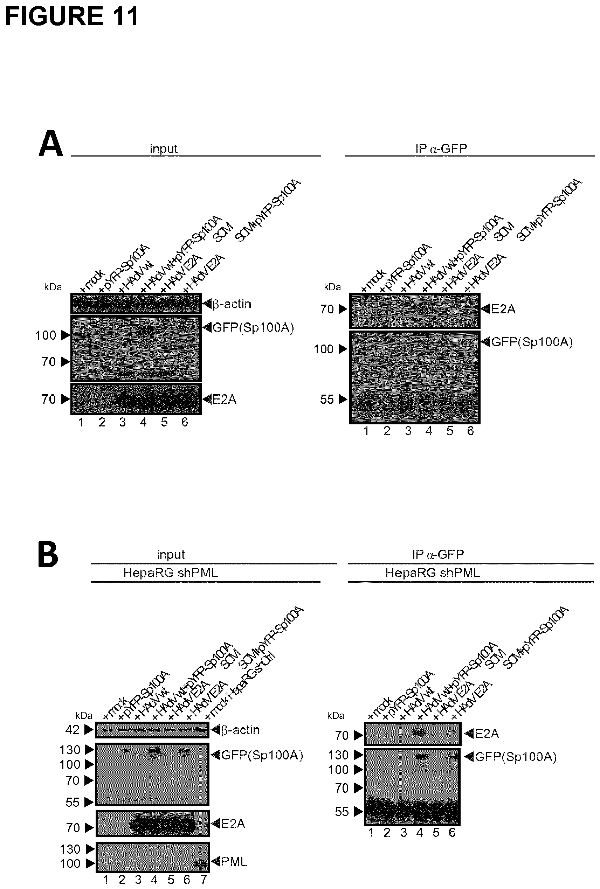

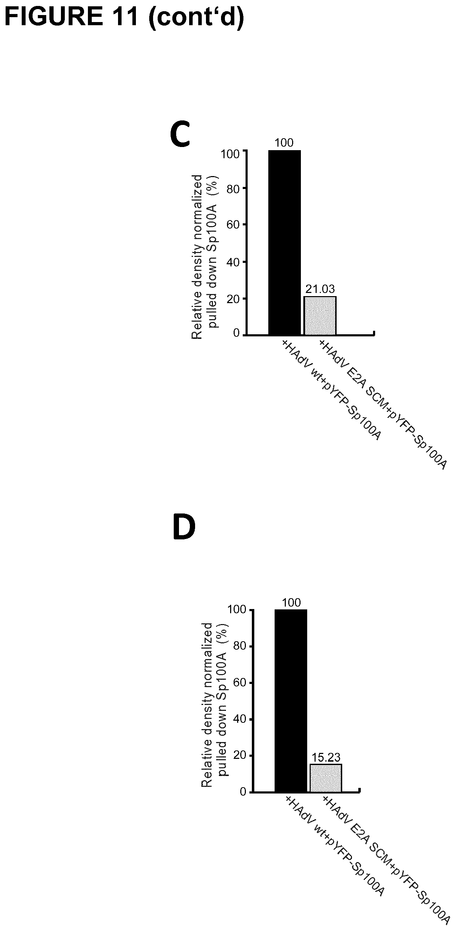

**Figure 11****: E2A SUMOylation promotes Sp100A-E2A interaction** (cf. Example 14). **(A)** HepaRG cells were transfected with pYFP-Sp100A (lane 2) and superinfected with HAdV wt (lane 4) and HAdV E2A SCM mutant (lane 6) at a multiplicity of 50 FFU/cell. The cells were harvested 24 h p.i. and whole-cell lysates were prepared. Immunoprecipitation analysis was performed using polyclonal rabbit antibody against GFP pAb260 (anti-GFP). Proteins were subjected to immunoblot analyses. Input levels of total-cell lysates and coprecipitated proteins were detected using mAb B6-8 (anti-E2A), pAb260 (anti-GFP) and mAb AC- 15 (anti-β-actin). Molecular weights in kDa are indicated on the left, relevant proteins on the right. **(B)** HepaRG shPML cells were transfected with pYFP-Sp100A (lane 2) and superinfected with HAdV wt (lane 4) and HAdV E2A SCM mutant (lane 6) at a multiplicity of 50 FFU/cell. The cells were harvested 24 h p.i. and whole-cell lysates were prepared. HepaRG shCtrl cells were used to verify PML depletion in HepaRG sh PML cells (left panel, lane 7). Immunoprecipitation analysis was performed using polyclonal rabbit antibody against GFP pAb260 (anti-GFP). After immunoblotting input levels of total-cell lysates and co-precipitated proteins were detected using mAb B6-8 (anti-E2A), pAb260 (anti-GFP), pAb NB100-59787 (anti-PML) and mAb AC-15 (anti-β-actin). Molecular weights in kDa are indicated on the left, relevant proteins on the right. **(C)** Densitometry analysis of E2A interaction with Sp100A in (A) above, (lanes 4 and 6), quantified with ImageJ (version 1.45s) software, normalized to the respective levels of immunoprecipitated YFP- Sp100A. **(D)** Densitometry analysis of E2A interaction with Sp100A in (B) above (lanes 4 and 6), quantified with ImageJ (version 1.45s) software, normalized to the respective levels of immunoprecipitated YFP-Sp100A.

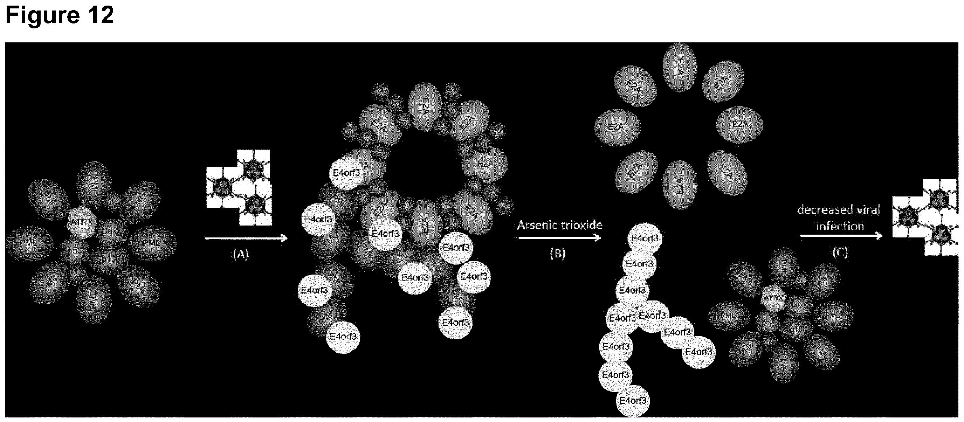

**Figure 12****: Mechanism of adenoviral replication and ATO treatment response** (cf. Example 15).

### DETAILED DESCRIPTION OF THE INVENTION

The present invention provides new means and methods for therapy and diagnosis of adenoviral infection. It has surprisingly been found that ATO acts as a potent inhibitor of adenovirus infection, in particular human adenovirus (HAdV) infection, due to inhibiting adenoviral replication by de-SUMOylation of early adenoviral protein E2A.

In one aspect, the present invention provides arsenic trioxide (As₂O₃) for use in the treatment or prevention of an adenovirus infection in a subject. On a more general basis, the present invention also provides arsenic trioxide (As₂O₃) for use in the treatment or prevention of a viral infection in a subject, wherein the virus is a DNA virus.

In one aspect, the present invention provides a method of treating or preventing an adenovirus infection in a subject, the method comprising administering a therapeutically effective amount of arsenic trioxide (As₂O₃) to a subject in need thereof. On a more general basis, the present invention provides a method of treating or preventing a viral infection in a subject, the method comprising administering a therapeutically effective amount of arsenic trioxide (As₂O₃) to a subject in need thereof, wherein the virus is a DNA virus.

The term "a therapeutically effective amount" as used herein refers to an amount of an agent which is effective, upon single or multiple dose administration to the subject, in providing a therapeutic benefit to the subject. In one embodiment, the therapeutic benefit is inhibiting virus activity. Within the scope of the present invention is a method for suppressing viral replication, the method comprising the step of administering to a subject in need thereof a therapeutically effective amount of arsenic trioxide alone or in combination with a compound having anti-viral activity. As further described herein, a "therapeutically effective amount" refers to an amount that will provide some alleviation, mitigation, and/or decrease in at least one clinical symptom associated with the viral infection, and/or disease or disorder described herein. Those skilled in the art will appreciate that the therapeutic effects need not be complete or curative, as long as some benefit is provided to the subject. Generally, the skilled artisan will recognize that the form of arsenic trioxide to be used should be therapeutically effective without unreasonable toxicity. The toxicity may be dependent upon the dose, the dosage form, the mode of administration and frequency of dosing.

The subject may suffer from an adenoviral infection, or may be at risk of contracting an adenoviral infection.

In various embodiments, the DNA virus may be any of a herpesvirus (including, but not limited to, HCMV, HSV, KSHV, EBV, VZV), HPV (human papilloma virus), HBV (hepatitis B virus), and polyomaviruses. As used herein, a DNA virus is a virus that has DNA as its genetic material and replicates using a DNA-dependent DNA polymerase. The nucleic acid is usually double-stranded DNA (dsDNA) but may also be single-stranded DNA (ssDNA).

As used herein, the term "arsenic trioxide" (As₂O₃/ATO) refers to a compound having the formula As₂O₃, and may also refer to derivatives thereof. Arsenic derivatives and uses thereof are described, for example, in Waxman et al. (Oncologist 6: 3-10, 2001; incorporated herein by reference). As used herein, "arsenic compound" refers to a pharmaceutically acceptable form of arsenic trioxide (As₂O₃) or its derivatives thereof. Accordingly, in one embodiment, arsenic trioxide, arsenic compounds or derivatives thereof are used in the treatment or prevention of a viral infection, in particular in the treatment or prevention of a DNA virus infection. In a preferred embodiment, the viral infection is an adenoviral infection, more preferably a human adenovirus (HAdV) infection.

As used herein, "arsenic trioxide" encompasses a pharmaceutically acceptable form of arsenic trioxide including salts, solutions, complexes, chelates and organic and inorganic compounds incorporating arsenic trioxide. The arsenic trioxide of the present invention can be synthesized or commercially purchased. For example, the arsenic trioxide can be prepared from well-known chemical techniques. (See for example, Kirk-Othmer, Encyclopedia of Chemical Technology, 4th ed. Vol. 3, pp. 633-655, John Wiley & Sons).

In various embodiments, the arsenic trioxide of the invention is dissolved in an aqueous solution of sodium hydroxide, with the pH adjusted to a physiologically acceptable range, e.g. about pH 6-8. Accordingly, the term "arsenic trioxide" encompasses a physiologically acceptable form of arsenic trioxide.

As used herein, "adenovirus" is a member of the family Adenoviridae and refers to all known genre including Genus Atadenovirus; Genus Aviadenovirus; Genus Ichtadenovirus; Genus Mastadenovirus (including all human adenoviruses); and Genus Siadenovirus. As used herein, "adenovirus" refers to all adenoviral species including known Human Adenovirus (HAdV) species, namely A, B, C, D, E, F, G and their individual serotypes including, but not limited to, types 1, 2, 3, 4, 4a, 5, 6, 7, 8, 9, 10, 11 (AdI 1A and AdI IP), 12, 13, 14, 15, 16, 17, 18, 19, 19a, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48 and 91. Each possibility represents a separate embodiment of the present invention. In a preferred embodiment, adenovirus as used herein is HAdV-C5.

The term "infection" as used herein may be considered as the invasion of an organism's body, or an organism's body tissues, by disease-causing agents, their multiplication, and the reaction of the host body, or host body tissues, to the infectious agents and the toxins they produce. The infectious agent may be a pathogen, in particular a virus, more specifically a DNA virus, or even more specifically an adenovirus, as described herein. Infectious disease, also known as transmissible disease or communicable disease, may be considered as illness resulting from an infection. Infections are caused by infectious agents (pathogens), including viruses as described above, as well as related agents such as viroids and prions. In preferred embodiments of the invention, infection as used herein is an adenoviral infection. More preferably, adenoviral infection may be HAdV-C5 infection.

As used herein, the terms "treatment", "treat", and "treating", may be considered to refer to reversing, alleviating, inhibiting the progress of a disease or disorder as described herein, or delaying, eliminating or reducing the incidence or onset of a disorder or disease as described herein, as compared to that which would occur in the absence of the measure taken. In some embodiments, treatment may be applied or administered after one or more symptoms have developed. In various embodiments, the treatment may be applied or administered in the absence of symptoms. For example, treatment may occur to a susceptible subject or individual prior to the onset of symptoms (*e.g*., in light of a history of symptoms and/or in light of genetic or other susceptibility factors). Treatment may also be continued after symptoms have resolved, for example to prevent or delay their recurrence. Accordingly, the term "treating" may further encompass prophylaxis or preemptive treatment, namely the prevention of infection and/or disease or condition as described herein in yet uninfected or infected asymptomatic subjects.

As used herein, the terms "prevention", "prophylactic", or "prophylaxis" means causing the clinical symptoms of a disease or condition not to develop, *i.e.,* inhibiting the onset of an infection and/or disease or condition as described herein in a subject that may be exposed to or predisposed to the infection, and/or disease or condition, but does not yet experience or display symptoms of the infection, and/or disease or condition. Preventative administration may be considered to mean that a compound or therapy according to the invention is administered to a subject prior to observation of symptoms and/or a suspected exposure to a causative agent of the condition, wherein the causative agent in the present invention is a virus as described herein, including DNA viruses, in particular adenoviruses as described herein. Generally, preventative administration may reduce (a) the likelihood that a subject that receives the treatment contracts the infection/develops the condition and/or (b) the duration and/or severity of symptoms in the event the subject contracts the infection/develops the condition.

"Subject," as used herein, refers to any organism or portion thereof to be administered a composition as described herein (e.g., arsenic trioxide, anti-viral agents and combinations or derivatives thereof). 'Portion' as used herein is intended to include tissues, cells and biological fluids or any other biological sample isolated from a subject, as well as tissues, cells and fluids present within a subject.

A subject may be a mammal, in particular an animal, such as a mouse, rat, rabbit, dog, cat, goat, pig, and horse. In a preferred embodiment, the subject is a human. In another embodiment, subject may be a bird, such as a pigeon, parrot, chicken, duck, goose, turkey and pheasant. In another embodiment, within the scope of the present invention is the treatment of newborns, infants, or toddlers that are infected with adenovirus or are at risk thereof, pregnant women who are infected with adenovirus or are at risk thereof, and transplantation recipients that are at risk of contracting a viral infection as described herein, in particular an adenoviral infection. Each possibility represents a separate embodiment of the present invention. In one embodiment, a subject as described herein is a subject suffering from an adenovirus infection. In another embodiment, subject as used herein is a subject at a risk of contracting an adenovirus infection. Accordingly, in a preferred embodiment, the viral infection which is treated or prevented by arsenic trioxide, arsenic compounds or derivatives thereof is an adenovirus infection in a human.

In various embodiments of the invention, the subject is suffering from a disease or disorder caused by and/or associated with an adenovirus infection. In other embodiments of the invention, the subject is at risk of contracting a disease or disorder caused by and/or associated with an adenovirus infection. Preferably, the subject is a subject suffering from, or at risk of contracting, a disease and/or disorder of the respiratory system. In another preferred embodiment, the subject is suffering from, or at risk of contracting, conjunctivitis. In another preferred embodiment, the subject is suffering from, or at risk of contracting, keratoconjunctivitis. In another preferred embodiment, the subject is suffering from, or at risk of contracting, gastroenteritis. In another preferred embodiment, the subject is suffering from, or at risk of contracting, pneumonia. In another preferred embodiment, the subject is suffering from, or at risk of contracting, hepatitis. In another preferred embodiment, the subject is suffering from, or at risk of contracting, encephalitis. In still another preferred embodiment, the subject is suffering from, or at risk of contracting, cystitis.

In various embodiments of the invention, the subject is an immunocompromised subject. Accordingly, in preferred embodiments, the treatment or prevention of the present invention is directed to an adenovirus infection in an immunocompromised subject. The term "immunodeficiency" or "immunosuppression" or "immunocompromised" as used herein may be considered to refer to a state in which the immune system's ability to fight an infection and/or an infectious disease or to mount an immune response is compromised or entirely absent. In this context, "immunocompromised" may refer to any reduction in number or function of any cells of the immune system, in particular T cells. The response by the immune system to an immunogen may be depressed as a consequence of certain diseases or pathological conditions. As described herein, an "immunocompromised" subject may be a subject that is more susceptible to a (pathological) infection or malignancy, against which a normal immune system would have otherwise provided sufficient response or protection. In various embodiments of the present invention, the term "immunocompromised" may refer to a subject incapable of developing, or unlikely to develop, a robust immune response, e.g., as a result of disease, malnutrition or immunosuppressive therapy. Those who can be considered to be immunocompromised include, but are not limited to, subjects with AIDS (or HIV positive), subjects with severe combined immune deficiency (SCID), and diabetics, subjects who have had transplants and who are taking immunosuppressive drugs, and those that are receiving chemotherapy or radiotherapy. Immunocompromised individuals may also include subjects with all forms of cancer, sickle cell anemia, cystic fibrosis, those who do not have a spleen, subjects with end stage kidney disease (dialysis), and those who have been taking corticosteroids on a frequent basis by pill or injection within the last year. Subjects with severe liver, lung or heart disease also may be immunocompromised. Furthermore, immune responses tend to be weaker in the elderly than in young humans. For this reason, the methods, uses and compositions of the present invention may also be particularly beneficial for elderly populations. Accordingly, in one embodiment the subject may be an elderly human (optionally wherein the elderly human is more than 50, 55, 60, 65, 70, and 75 or 80 years old). In further embodiments, the subject may be a human adult, which may be a human of ≥18 years of age. In further embodiments, the subject may be an elderly human or person aged 60, 62, 65, 67, or 70 years and over. In still further embodiments, the subject may be a younger adult (e.g., between 18 and 50 years of age). In other embodiment of the invention, the subject may be an infant human such as a child more than 6 months old, especially children 6 to 23 months of age. Optionally the subject may be a toddler such as a child between 24 months and 3 years of age. Accordingly, in various embodiments of the invention, arsenic trioxide is used in the treatment or prevention of an adenoviral infection in a subject that is incapable of developing a normal immune response.

In preferred embodiments of the invention, immunocompromised subjects are recipient subjects. Recipient subject, as used herein, refers to a subject that may receive an organ transplant, specifically a solid-organ transplant. Subjects with an organ transplant are also in need of treatment to suppress the immune system in order to suppress or prevent organ transplant rejection. An "organ transplant" includes transferring or "transplanting" an internal organ (e.g., heart, lung, kidney, liver, pancreas, stomach, large intestine and small intestine) or external organ (e.g. skin) from a donor to a recipient, wherein the donor is genetically distinct from the individual or animal who has received the transplant. An "organ transplant" also includes cross species transplants. Recipient subject, as used herein, also refers to a subject that is receiving a "stem cell transplant", which may be considered to refer to a composition comprising stem cells, wherein the composition is suitable for administration by transplantation into a recipient subject. The stem cell transplant may advantageously be obtained from a (tissue) biopsy, such as peripheral blood, umbilical cord blood or bone marrow. Collection of bone marrow or peripheral blood for use in autologous or allogeneic stem cell transplantation therapies is common practice and methods to collect bone marrow or peripheral blood biopsies are well known in the art. Recipients of the stem cell transplants of the invention can be autologous or allogeneic. In a preferred embodiment, the cell transplant of the invention is an autologous transplant. In another embodiment, the stem cell transplant of the invention is an allogeneic transplant. Hematopoietic stem cell transplant refers to the collection and transplantation of hematopoietic stem cells.

In other embodiments, immunocompromised subjects may be subjects suffering from cancer and/or an additional chronic infection. It is well known to those of skill in the art that the immune system of cancer patients often is impaired due to the disease and also due to immunosuppressive therapies such as chemotherapy and radiation therapy.

In accordance with the above, the subject of the present invention may in particular be an immunocompromised (solid) organ transplant recipient. In other embodiments, the subject may in particular be an immunocompromised stem cell transplant recipient.

In other preferred embodiments, the subject may be an immunocompromised subject suffering from cancer. The subject suffering from cancer may in particular be immunocompromised due to cancer therapy received, including, but not limited to, cancer radiotherapy and/or cancer chemotherapy.

Cancer as used herein, refers to all types of cancers or neoplasms or malignant tumors. The term "cancer" includes solid tumors and haematological cancers, and also includes cancer metastasis. Representative types of cancer include cervical cancer, uterine cancer, ovarian cancer, kidney cancer, gallbladder cancer, liver cancer, head and neck cancer, squamous cell carcinoma, gastrointestinal cancer, breast cancer, pancreatic cancer, prostate cancer, testicular cancer, lung cancer, non-small cell lung cancer, non-Hodgkin's lymphoma, multiple myeloma, leukemia (such as acute lymphocytic leukemia, chronic lymphocytic leukemia, acute myelogenous leukemia, and chronic myelogenous leukemia), brain cancer (e.g. astrocytoma, glioblastoma, medulloblastoma), neuroblastoma, sarcomas, colon cancer, rectum cancer, stomach cancer, anal cancer, bladder cancer, cancer of the urinary tract, thyroid cancer, renal cancer, carcinoma, endometrial cancer, plasmacytoma, lymphomas, retinoblastoma, Wilm's tumor, Ewing sarcoma, melanoma and other skin cancers. Each possibility represents a separate embodiment of the present invention.

In one aspect, the arsenic trioxide is for use in a method of preventing and/or inhibiting recurrent, persistent, and/or *de novo* viral infection, wherein the virus is a DNA virus. Preferably, the arsenic trioxide is for use in a method of preventing and/or inhibiting recurrent, persistent, and/or *de novo* adenoviral infection.

"Inhibition" as used herein is inhibition of viral cell entry or early steps of the viral life cycle (such as reverse transcription or integration into the host cell genome) by a candidate inhibitor where the indicator cell survives. Inhibition of these early steps of the viral life cycle is therapeutically superior to inhibition of later steps like viral assembly or budding since at that late stage the viral genome has already integrated into the host cell genome and is thus inevitably conserved. In the present invention, the inhibitor is arsenic trioxide, or an arsenic compound, and may encompass derivatives thereof.

In an embodiment of the invention, arsenic trioxide is inhibiting adenovirus infection by antagonizing (productive) adenovirus infection cycles. In various embodiments of the invention, ATO does not cause cellular cytotoxicity. Figure 2B shows highly efficient inhibition of adenovirus infection with ATO after 24 and 48h p.i. (post infection). Accordingly, in an embodiment of the invention, ATO, preferably ATO at 2µM, inhibits adenoviral infection by up to 50% after 24h p.i. In another embodiment of the invention, ATO, preferably ATO at 2µM, inhibits adenoviral infection by more than 50% after 48h p.i. In another embodiment of the invention, ATO, preferably ATO at 4µM, inhibits adenoviral infection by up to 75% after 24h p.i. In yet another embodiment of the invention, ATO, preferably ATO at 4µM, inhibits adenoviral infection by more than 75% after 48h p.i.

In other embodiments of the invention, arsenic trioxide is inhibiting adenovirus infection by reducing the number of cells expressing viral E2A protein. In still other embodiments, arsenic trioxide is inhibiting adenovirus infection by reducing the number of cells expressing late adenovirus capsid protein. Figure 1A shows that the number of E2A and capsid expressing cells was counted and a reduction of up to 40% and up to 80% in cells expressing E2A and capsid, was observed, respectively.

In other embodiments of the invention, arsenic trioxide is inhibiting adenovirus infection by reconstitution of PML-NB antiviral activity.

In other embodiments of the invention, arsenic trioxide is inhibiting adenovirus infection by blocking efficient adenovirus protein expression. In a preferred embodiment of the invention, arsenic trioxide is inhibiting adenovirus infection by reducing the expression of E2A protein. Figure 2A and 3C show reduction in E2A protein expression upon ATO treatment. In one embodiment, ATO, preferably ATO at 8µM, inhibits adenoviral infection by completely blocking E2A protein expression at 24h p.i. In another embodiment, ATO, preferably ATO at 4µM, inhibits adenoviral infection by reducing E2A protein expression by more than 50% at 24h p.i. In another preferred embodiment, ATO, preferably ATO at 8µM, inhibits adenoviral infection by completely blocking E2A protein expression at 48h p.i. In another embodiment, ATO, preferably ATO at 8µM, inhibits adenoviral infection by reducing E2A protein expression by up to 60% at 48h p.i..

In another embodiment, arsenic trioxide is inhibiting adenovirus infection by interfering with efficient virus particle assembly. Figure 2A and 3D show reduction in capsid protein concentrations upon ATO treatment. In a preferred embodiment, arsenic trioxide is inhibiting adenovirus infection by reducing the expression of late capsid protein. In one embodiment, ATO, preferably ATO at 4µM, inhibits adenoviral infection by complete loss of capsid expression at 48h p.i. In another embodiment, ATO, preferably ATO at 2µM, inhibits adenoviral infection by reducing capsid expression by 40% at 48h p.i..

In other embodiments of the invention, arsenic trioxide is inhibiting adenovirus infection by reducing the virus yield. Figure 1B shows virus progeny production upon ATO treatment, where a reduction of 80% in virus yield was observed. Accordingly, in preferred embodiments of the invention, ATO, preferably ATO at 2µM, inhibits adenoviral infection by completely abolishing virus yield. In other preferred embodiments, ATO, preferably ATO at 1µM, inhibits adenoviral infection the by 80% reduction of virus yield.

The term *'de novo'* as used herein means "from the beginning" or "anew". In various embodiments of the invention, the viral infection is a *de novo* viral infection, more specifically a *de novo* adenoviral infection.

The term "recurrent viral infection" as used herein is an infection following recovery from or superimposed on infection of the same type. Accordingly, in various embodiments of the invention, the viral infection is a recurrent viral infection, more specifically a recurrent viral infection.

The term "persistent infection" refers to those infections that, in contrast to acute infections, are not effectively cleared by therapy and/or the induction of a host immune response. During such persistent infections, the infectious agent and the immune response reach equilibrium such that the infected subject remains infectious over a long period of time without necessarily showing symptoms. Accordingly, in various embodiments of the invention, the viral infection is a persistent viral infection, more specifically a persistent adenoviral infection. A persistent infection as described herein may involve stages of both silent and productive infection without rapidly killing or even producing excessive damage of the host cells. A persistent infection may include, for example, latent, chronic and slow infections. Each possibility represents a separate embodiment of the present invention.

In one aspect of the invention, arsenic trioxide is for use in a method of preventing and/or inhibiting replication of adenovirus. As used herein, viral replication may be considered as the formation of biological viruses during the infection process in the host cell of a subject of the present invention. It is understood to a skilled person that the virus must first get into the cell before viral replication can occur. Through the generation of abundant copies of its genome and packaging these copies, the virus continues infecting new hosts or host cells, respectively. Replication between viruses is greatly varied and depends on the type of genes involved in them. Most DNA viruses assemble in the nucleus while most RNA viruses develop solely in cytoplasm. Viruses multiply only in living cells. The host cell must provide the energy and synthetic machinery and the low molecular-weight precursors for the synthesis of viral proteins and nucleic acids. In a preferred embodiment, viral replication, as used herein, is a replication of a DNA virus. In an even preferred embodiment, viral replication refers to adenovirus replication. In various embodiments, the viral replication is a *de novo* adenoviral replication. In other embodiments, the viral replication is a recurrent adenoviral replication. In still other embodiments, the viral replication is a persistent adenoviral replication.

Adenoviral genomes have been observed to be juxtaposed to PML-NBs, and the formation of replication and transcription domains (RCs; replication centers) often takes place in close proximity to PML-NBs. In the present invention, ATO may be considered to reduce the number of adenovirus replication centers. ATO has been shown to reduce the number of PML-NBs. Figure 4 shows these results.

In another embodiment, arsenic trioxide is used in method for the treatment, prevention and/or inhibition of adenovirus infection by reducing the adenoviral DNA synthesis. ATO has been shown to reduce adenoviral DNA replication. Figure 3A shows blocking of adenoviral DNA replication by ATO treatment. Accordingly, in various embodiments, ATO, preferably ATO at 1µM, blocks adenoviral DNA replication by up to 60% reduction of viral DNA. In other embodiments, ATO, preferably ATO at 2µM, blocks adenoviral DNA replication by up to 80% reduction of viral DNA.

In one aspect, provided is arsenic trioxide for use in reducing the adenoviral load. The term "viral load" as used herein, also known as viral burden, viral titer or viral titer, may be considered as a numerical expression of the quantity of virus in a given volume. Quantitative polymerase chain reaction (PCR) can be used to detect adenovirus genome and to determine viral load. Tracking viral load can be used to monitor therapy during viral infections. Viral load as used herein preferably means adenoviral load, and arsenic trioxide can be used in a method for treating adenoviral infection by reducing the adenoviral load in a subject. Figure 2D shows the real-time kinetic data of adenoviral expression at different ATO concentrations. Accordingly, in various embodiments, ATO reduces the viral load (over time) with a four-fold reduction at 60h p.i.

In another embodiment of the invention, arsenic trioxide is inhibiting adenovirus infection by blocking efficient adenovirus gene expression. Figure 3B shows gene expression of early (E1A) and late (Hexon) adenoviral genes. Accordingly, in various embodiments, ATO directly affects early and late viral mRNA production. In other embodiments, ATO, preferably ATO at 1µM, leads to a reduction of E1A and Hexon viral transcripts by up to 40%. In another embodiment, ATO, preferably ATO at 2µM, leads to a reduction of E1A viral transcripts by up to 80%. In another embodiment, ATO, preferably ATO at 2µM, leads to a reduction of E1A viral transcripts by up to 60%.

It will be understood by those skilled in the art that the compositions and methods of the present invention have utility for treating not only (adeno)viral infections themselves, but also preventing or treating diseases and disorders engendered by (adeno)viral infections. Accordingly, in various embodiments of the invention, the subject is a subject suffering from, or at risk of contracting, a disease or disorder associated with, or caused by, an adenovirus infection. Preferably, a disease or disorder engendered by adenovirus infection may include a disease or disorder of the respiratory system, conjunctivitis, keratoconjunctivitis, gastroenteritis, pneumonia, hepatitis, encephalitis, and/or cystitis, without being limited thereto. Each possibility represents a separate embodiment of the present invention.

As described herein, diseases or disorders of the respiratory system include, but are not limited to, asthma, respiratory allergy, pneumonia, bronchitis, rhinitis, sinusitis, tracheitis, pharyngitis, croup, and otitis. The disease or disorder of the respiratory system may a chronic or an acute disease or disorder of the respiratory system. As further described herein, gastroenteritis refers to inflammation of the gastrointestinal tract. As further described herein, pneumonia refers to an infection and/or inflammation of the lungs. As further described herein, hepatitis may be considered to refer to any clinically significant inflammation of the liver or biliary system, regardless of etiology. Hepatitis as used herein includes "acute hepatitis" referring to any short term (less than six months) or initial-stage liver inflammation, such as the initial stages of hepatitis virus infection. Hepatitis as used herein also includes "chronic hepatitis" including any inflammation of the liver persisting six months or longer. Cystitis as used herein includes interstitial cystitis and may be considered to refer to chronic pelvic pain disorders, a condition resulting in recurring discomfort or pain in the bladder and the surrounding pelvic region. Cystitis as used herein also includes "hemorrhagic cystitis" and refers to bladder inflammation as the result of a chemical or other traumatic insult to the bladder.

As further described herein, a viral infection according to the present invention may be a chronic or an acute viral infection, in particular a chronic or an acute adenoviral infection. Tumorigenic potential has been attributed to the capacity of some adenoviruses to turn off the expression of genes of the major histocompatibility complex and thus to allow the transformed cells to overcome host defenses and grow into solid tumors. Most of the adenovirus-induced tumors, tumor cell lines, and transformed cell lines carry one or several copies of the viral genome integrated into the chromosomes. The tumor or transformed state is also associated with the differential expression of the integrated viral genes. The early viral genes are often the predominant genes expressed. It is thought that the E1 region of the viral genome is particularly important in eliciting the transformed state. The few studies on this topic have reported occasional changes of oncogene activity, particularly for the myc gene. Moreover, E1 proteins can bind tightly to the product of the retinoblastoma (RB) or the p53 gene, which are considered to be anti-oncogenes. It has been suggested that the fixation of the anti-oncogene products by E1 proteins might contribute to the transformation of cells. The interplay of several viral and cellular factors may eventually alter the cellular growth control and weaken or overcome the host defenses in such a way that an adenovirus-transformed rodent cell can grow into a solid tumor. Accordingly, in a specific embodiment, a disease caused by adenovirus infection may be cancer. Hence, in yet another specific embodiment of the invention, arsenic trioxide may be used in a method for treating or preventing cancer caused by an adenovirus, or cancer associated with an adenoviral infection. In still other specific embodiments, the present invention excludes the use of arsenic trioxide, or arsenic compounds or derivatives thereof as described herein, in a method for treating or preventing cancer in a subject, in particular treating or preventing in a subject cancer caused by an adenovirus, or cancer associated with an adenoviral infection.

In another aspect of the invention, arsenic trioxide is used for lessening the severity of clinical symptoms in a subject suffering from a disease or disorder associated with, or caused by, a viral infection as described herein, in particular an adenoviral infection as described herein. As used herein, the term "reduction of clinical symptoms" or "lessening of the severity of clinical symptoms" may be considered to mean, but is not limited thereto, reducing the severity of any clinical signs or symptoms that are present in a subject suffering from a disease or disorder associated with, or caused by, a viral infection as described herein, in comparison to a subject not suffering the disease or disorder associated with, or caused by, the viral infection. For example, it may refer to any reduction of pathogen load, pathogen shedding, reduction in pathogen transmission, or reduction of any clinical sign symptomatic of the pathogen. In a preferred embodiment, pathogen as used herein is adenovirus. Preferably, the clinical signs are reduced in one or more subjects receiving the therapeutic treatment of the present invention by at least 10% in comparison to a subject suffering from the disease or disorder associated with, or caused by, a viral infection as described herein not receiving the therapeutic treatment according to the present invention. More preferably, clinical signs are reduced in subjects receiving the therapeutic treatment of the present invention by at least 20%, still more preferably by at least 30%, or at least 40%, and even more preferably by at least 50%.

The "lessening of the severity of clinical symptoms", and similar phrases, may also mean a partial or complete response against an infection and/or disease or condition generated by administration of one or more therapeutic compositions of the invention, or a combination thereof, that results in fewer deleterious effects than would be expected in a subject that has been exposed to infection, and/or disease or condition.

In another aspect of the present invention, the ATO therapy described herein further comprises treatment of the subject with an anti-viral agent, which is not arsenic trioxide, in particular with an anti-adenoviral agent, which is not arsenic trioxide. The term "anti-(adeno) viral agent" (which is not ATO) as used herein refers to a chemical material or compound which, when administered to a subject or an organism (human or animal), kills viruses, blocks virus effects on the host cells, prevents infection with a virus, and/or suppresses viral replication and, hence, inhibits the capability of the virus to infect, multiply, reproduce, or alter host physiology and biochemistry. In particular, the "anti-(adeno)viral agent" (which is not ATO) may encompass a single biological or a biological chemical compound, or a combination of biological compounds, that may be required to cause a desirable therapeutic effect. Such antiviral agents may be useful in the early stages of some viral infections, or to prevent reoccurrences or reactivation in chronic infections. Most antiviral agents exert their effects only during a certain stage of viral replication. Antivirals are designed to target and disable viral proteins, or parts of proteins that are specific to the virus, and ideally these viral targets are as distinct as possible from any proteins, or parts of proteins, in humans in order to reduce unacceptable side effects. Antiviral agents have been developed to disable viral replication at different steps of the viral life cycle including, but not limited to, viral entry into the host cell, replication of the viral genome, activation of viral protein, and release of new virus particles. For example, antiviral agents may inhibit the ability of a virus to enter a host cell by preventing the virus from binding to receptors on the host cell that are required for entry, or blocking the virus uncoating process inside the host cell such that the virus cannot release its contents. Additionally, antiviral agents may target the processes that synthesize viral components after a virus invades a host cell (e.g., adenovirus DNA polymerase). There are several classes of antiviral agents that block viral synthesis including ribonucleotide reductase inhibitors, nucleotide analogs, nucleoside analogs, antisense drugs, ribozymes, and protease inhibitors. Finally, antiviral agents, such as interferon drugs, may function to prevent the release of newly packaged virus particles from the infected host cell thereby blocking the final stage of the viral life cycle.

Anti-(adeno)viral agents (which are not ATO) as used herein may block (adeno)virus replication at any of the stages in the (adeno)virus life cycle, and may be effective treatments for preventing (adeno)virus related diseases or disorders.

A number of prescription agents have been shown to have antiviral effects, which may be used in combination with the ATO therapy of the present invention. In some embodiments, antiviral agents include, without limitation, Abacavir, Acyclovir, Amantadine, Amprenavir, Cidofovir, Didanosine, Darunavir, Delavirdine, Didox, Efavirenz, Emtricitabine, Enfuvirtide, Entecavir, Famciclovir, Foscarnet, Gancyclovir, Indinavir, Lamivudine, Nevirapine, Nelfinavir, Oseltamivir, Palivizumab,Pleconaril, Ribavirin, Rimantadine, Ritonavir, Saquinavir, Stavudine, Tridox, Valacyclovir, Vidarabine, Zalcitabine, Zanamivir, and Zidovudine.

Accordingly, in certain aspects and embodiments, the present invention provides a combination therapy comprising the ATO therapy according to the present invention and at least one other anti-(adeno)viral drug. Should the ATO therapy of the present invention be administered as a combination therapy with one or more additional therapeutic agents (e.g., one or more other anti-(adeno)viral agents not being ATO), the treatment may take place sequentially in any order, simultaneously, or as a combination thereof. For example, ATO therapy of the invention can take place prior to, after, or at the same time as administration of the additional therapeutic agent(s). In addition, the additional agent(s) can be administered during the period of ATO therapy of the invention, but does not need to occur over the entire treatment period. In another embodiment, the treatment regimen may include pre-treatment with one agent, followed by the addition of the other agent or agents. Alternating sequences of administration are also contemplated herein. The therapeutic efficacy of a combination therapy as described herein may be considered at least additive.

As used herein, "administration" (of a therapy of the present invention) can be accomplished by or may include, e.g., oral, rectal, transdermal, topical, transmucosal, especially transnasal, intestinal or parenteral delivery, including intramuscular, subcutaneous and intramedullary injections, as well as intrathecal, direct intraventricular, intravenous, intraperitoneal, intranasal, intraarterial, intravesicle (into the bladder) or intraocular injections. For oral administration, the pharmaceutical preparation may be in liquid form, e.g., solutions, syrups or suspensions, or may be presented as a drug product for reconstitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, or fractionated vegetable oils); and preservatives (e.g., methyl or propyl-p-hydroxybenzoates or sorbic acid. Preparations for oral administration may be suitably formulated to give controlled release of the active compound. Each possibility represents a separate embodiment of the present invention.

The ATO, or arsenic compounds, of the invention may be formulated into pharmaceutical preparations for administration according to the present invention. The pharmaceutical preparations or compositions of the present invention comprise ATO, or a pharmaceutically acceptable salt thereof, or a derivative thereof, as the active ingredient, and may also contain a pharmaceutically acceptable carrier, and optionally other therapeutic ingredients, for example non-ATO anti-virals as described elsewhere herein. The term "pharmaceutically acceptable salts" refers to salts prepared from pharmaceutically acceptable non-toxic acids and bases, including inorganic and organic acids and bases. If the complex is water-soluble, then it may be formulated in an appropriate buffer, for example, phosphate buffered saline or other physiologically compatible solutions. Alternatively, if the resulting complex has poor solubility in aqueous solvents, then it may be formulated with a non-ionic surfactant such as Tween, polyethylene glycol or glycerol. The pharmaceutical compositions may take the form of, for example, tablets or capsules prepared by conventional means with pharmaceutically acceptable excipients such as binding agents (e.g., pregelatinized maize starch, polyvinyl pyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well-known in the art. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use. As will be appreciated by those of ordinary skill in the art, the magnitude of a therapeutic dose of an arsenic compound in the acute or chronic management of an (adeno)viral infection will vary with the severity of the condition to be treated and the route of administration. The dose, and perhaps dose frequency, will also vary according to the age, body weight, condition and response of the individual patient. In general, the total daily dose ranges for the conditions described herein are generally from about 10 µg to about 200 mg administered in divided doses administered parenterally or orally or topically. A total daily dose may be from about 0.1 mg to about 70 mg of the active ingredient, preferably from about 0.1 mg/kg/d to 0.5 mg/kg/d, more preferably from about 0.1 mg/kg/d to 0.3 mg/kg/d, still more preferably from about 0.1 mg/kg/d to 0.2 mg/kg/d, and even more preferred at a dose of 0.15 mg/kg/d.

In addition, the arsenic trioxide carrier could be delivered via charged and uncharged matrices used as drug delivery devices such as cellulose acetate membranes, also through targeted delivery systems such as fusogenic liposomes attached to antibodies or specific antigens In practical use, an arsenic compound can be combined as the active ingredient in intimate admixture with a pharmaceutical carrier according to conventional pharmaceutical compounding techniques. The carrier may take a wide variety of forms depending on the form of preparation desired for administration, *e.g*., oral or parenteral (including tablets, capsules, powders, intravenous injections or infusions). In preparing the compositions for oral dosage form any of the usual pharmaceutical media may be employed, *e.g*., water, glycols, oils, alcohols, flavoring agents, preservatives, coloring agents, and the like; in the case of oral liquid preparations, e.g., suspensions, solutions, elixirs, liposomes and aerosols; starches, sugars, microcrystalline cellulose, diluents, granulating agents, lubricants, binders, disintegrating agents, and the like in the case of oral solid preparations, *e.g*., powders, capsules, and tablets. In preparing the compositions for parenteral dosage form, such as intravenous injection or infusion, similar pharmaceutical media may be employed, *e.g*., water, glycols, oils, buffers, sugar, preservatives and the like know to those skilled in the art.

Auxiliary components such as tablet disintegrants, solubilisers, preservatives, antioxidants, surfactants, viscosity enhancers, coloring agents, flavoring agents, pH modifiers, sweeteners or taste-masking agents may also be incorporated into the composition.

The administration schedule can be taken once-daily, twice-daily, thrice-daily, once-weekly, twice-weekly, thrice-weekly, once-monthly, twice-monthly, thrice-monthly, or any other administration schedule known to those of skill in the art. In addition, the administration can be continuous, i.e., every day, or intermittently. The terms "intermittent" or "intermittently" as used herein means stopping and starting at either regular or irregular intervals. For example, intermittent administration can be administration in one to six days per week or it may mean administration in cycles (e.g. daily administration for two to eight consecutive weeks, then a rest period with no administration for up to one week) or it may mean administration on alternate days.

In a particularly preferred embodiment, arsenic trioxide is to be administered in the therapy according to the present invention at a low dose, which may be considered as a dose of 0.15-0.16 mg/kg/d or less, more specifically, 0.15 mg/kg/d or less. The administration regimen can be determined by a skilled artisan depending on the infection and the severity of the condition, the patient population, age, weight etc., and can be determined by standard clinical techniques. The precise dose to be employed may also depend on the route of administration, and the progression of the infection, and/or disease or disorder, and should be decided according to the judgment of the practitioner. Given the potential toxicity of arsenic compounds, the dosage and the duration of the treatment are determined in an appropriate manner, according to the seriousness of the infection, and/or disease or disorder and the long-lasting nature, or otherwise, of the recovery, it being possible for the treatment to last from a few days to a few months until complete or at least partial recovery is achieved. The formulation is typically administered daily or less frequently. Several successive treatments may be carried out, of the order of one month apart.

As described herein, a "low dose" or "low dosage" may be considered to mean a dosage of at least 5% less (e.g., at least 5%, 10%, 20%, 25%, 30%,40%, 50%, 60%, 70%, 80%, 90%, 91%, 92%, 93%, 94%,95%, 96%, 97%, 98%, or 99% less) than the standard recommended dosage or lowest standard recommended dosage of a particular agent, *e.g*., a therapeutic agent formulated for a given route of administration for treatment of any infection, and/or disease or condition. For example, a low dosage of an agent formulated for oral administration may differ from a low dosage of the agent formulated for intravenous administration. In some instances, a low dosage of an agent may be selected to be a nontoxic dosage of the agent. In some instances, a low dosage may be selected as a dosage that minimizes particular side effects of an agent, but which may still retain some side effects. For example, a dosage may be selected that minimizes or eliminates side effects that can lead to significant mortality or severe illness among subjects while still permitting more tolerable side effects, such as headache. In some instances, a low dose of arsenic trioxide may be considered as a dose of about 2 mg/kg body weight (per day) or less (e.g., about 0.1, 0.15, 0.16, 0.2, 0.3, 0.4, 0.5, 0.6, 0.7, 0.8, 0.9, 1.0, 1.25, 1.5, 1.75, or 2 mg/kg/d or less). In other instances, a low dose of arsenic trioxide may be a dose between about 0.5 mg/kg and about 5 mg/kg body weight per day (e.g., about 0.5, 0.6, 0.7, 0.8, 0.9, 1, 2, 3, 4, or 5, mg/kg/d or less). The effective amount intended to be administered daily may be a dose of 0.01 to 0.5mg/kg (of body weight), preferably 0.05 to 0.5 mg/kg, even more preferably 0.05 to 0.30 mg/kg, for example 0.10 to 0.30 mg/kg, or more precisely 0.10 to 0.20 mg/kg, even more preferably 0.05 to 0.15 mg/kg. In yet another embodiment, an effective amount of arsenic trioxide when used in a biological sample obtained from a subject thereof, is in molar concentration. In a preferred embodiment, arsenic trioxide is used in concentrations of up to 8.0 µM. In other preferred embodiments, arsenic trioxide is used in concentrations of up to 7.0 µM or up to 6.0 µM. In other preferred embodiments, arsenic trioxide is used in concentrations of up to 5.0 µM. In other preferred embodiments, arsenic trioxide is used in concentrations of up to 4.0 µM. In other preferred embodiments, arsenic trioxide is used in concentrations of up to 3.0 µM. In other preferred embodiments, arsenic trioxide is used in concentrations of up to 2.0 µM. In other preferred embodiments, arsenic trioxide is used in concentrations of up to 1.0 µM. In other preferred embodiments, arsenic trioxide is used in concentrations between 1.0 µM to 8.0 µM.

In another embodiment, an effective amount of arsenic trioxide leads to peak serum levels of 5.0 to 8.0 µM in a subject, preferably between 5.10 to 7.90 µM, more preferably between, 5.20 to 7.80 µM, even more preferably between 5.30 to 7.70 µM, even more preferably between 5.40 to 7.60 µM, even more preferably between 5.50 to 7.50 µM, more preferably between 5.45 to 7.40 µM, and most preferably between 5.54 to 7.30 µM.

The present invention also provides a kit for carrying out the therapeutic regimens of the invention. Such kits comprise in one or more containers therapeutically effective amounts of the arsenic compounds in pharmaceutically acceptable form. The arsenic compound in a vial of a kit of the invention may be in the form of a pharmaceutically acceptable solution, e.g., in combination with sterile saline, dextrose solution, or buffered solution, or other pharmaceutically acceptable sterile fluid. Alternatively, the complex may be lyophilized or desiccated; in this instance, the kit optionally further comprises in a container a pharmaceutically acceptable solution (e.g., saline, dextrose solution, etc.), preferably sterile, to reconstitute the complex to form a solution for injection purposes. In another embodiment, a kit of the invention further comprises a needle or syringe, preferably packaged in sterile form, for injecting the complex, and/or a packaged alcohol pad. Instructions are optionally included for administration of arsenic compounds by a clinician or by the patient.

In a further aspect, the present invention provides a method of detecting an adenovirus infection in a subject, comprising detecting SUMOylation of E2A protein, preferably detecting E2A SUMO conjugates, in a sample obtained from the subject. The terms "E2A SUMO conjugates" and "E2A SUMO moieties" may be used herein interchangeably. SUMO means Small Ubiquitin-like Modifier, or Small Ubiquitin-related Modifier. As used herein, the term SUMO protein is used, in general, to mean any SUMO protein, *i.e.,* SUMO 1-4 from any species, preferably human. In preferred embodiments, the SUMO protein is SUMO-2. For example, in various embodiments, the SUMO protein as used herein may have at least 90%, 95% or 99% identity with the sequence of SEQ ID NO:1, SEQ ID NO:2, SEQ ID NO:3, and/or SEQ ID NO:4. The SUMO proteins may have 90%, 95%, or 99% sequence identity with the mature form of the protein, polypeptide or a pro-peptide thereof.

SEQ ID NO: 1 – SUMO-1
MSDQEAKPSTEDLGDKKEGEYIKLKVIGQDSSEIHFKVKMTTHLKKLKESYCQRQGVPMNS
LRFLFEGQRIADNHTPKELGMEEEDVIEVYQEQTGGHSVT

SEQ ID NO: 2 – SUMO-2
MADEKPKEGVKTENNDHINLKVAGQDGSWQFKIKRHTPLSKLMKAYCERQGLSMRQIRFR
FDGQPINETDTPAQLEMEDEDTIDVFQQQTGGVY

SEQ ID NO: 3 – SUMO-3
MSEEKPKEGVKTENDHINLKVAGQDGSWQFKIKRHTPLSKLMKAYCERQGLSMRQIRFRF
DGQPINETDTPAQLEMEDEDTIDVFQQQTGGVPESSLAGHSF

SEQ ID NO: 4- SUMO-4
MANEKPTEEVKTENNNHINLKVAGQDGSWQFKIKRQTPLSKLMKAYCEPRGLSMKQIRFRF
GGQPISGTDKPAQLEMEDEDTIDVFQQPTGGVY

The mature form of SEQ ID NO: 1 is amino acid residues 1-97. The mature form of SEQ ID NO: 2 is amino acid residues 1-93. The mature form of SEQ ID NO: 3 is amino acid residues 1-92. The mature form of SEQ ID NO: 4 is amino acid residues 1-93.

The present invention relates to the finding that SUMOylation of the early adenoviral protein E2A is prevented that directly regulates the replication of the adenovirus. The inventors have found that adenoviral E2A is a novel target of the host SUMO conjugation machinery to enhance viral replication.

The inventors found that adenoviral E2A protein undergoes the process of SUMOylation by the interaction with the SUMO protein at its SUMO conjugation motifs (SCM) as shown in Figure 5B. By mutating these SCMs, the inventors surprisingly found that E2A wt protein binds to SUMO more efficiently than E2A SCM mutated one as shown in Figure 5C. The inventors also found that E2A SUMOylation controls its binding to PML-NBs. As shown in Figure 7, E2A SUMOylation regulates E2A/PML interaction during adenoviral infection. The inventors found that E2A SUMOylation affects the number of adenoviral RCs. The number of adenoviral RCs was reduced by 26.9% in E2A SCM mutant virus as shown in Figure 8. The inventors found that E2A SUMOylation is beneficial for adenoviral replication and affects adenoviral progeny as shown in Figure 9A. E2A SCM mutant virus progeny was reduced by 62% after 24h, 41% after 48h and 50% after 72h. Further, as shown in Figure 9B, the inventors found that the late protein expression and Hexon mRNA synthesis in infected cells was reduced with E2A SCM mutant virus. Finally, the inventors surprisingly found that ATO treatment leads to the decrease in E2A SUMOylation as shown in Figure 10B. Accordingly, in a preferred embodiment, ATO decreases the presence of E2A SUMO moieties in a subject or portion thereof by up to 70%. Thus, detection of E2A SUMO moieties can be an important tool for determining adenovirus infection and replication in a subject. To that end, one aspect of the invention is directed to diagnostic methods, where the methods comprise determining SUMOylation of the E2A protein in a subject or portion thereof, wherein, SUMOylation of E2A indicates replicating adenoviral infection. Accordingly, in various embodiments, arsenic trioxide is used in a method for the treatment, prevention and/or inhibition of adenovirus infection by preventing and/or inhibiting adenoviral replication by reducing, preventing and/or inhibiting SUMOylation of adenoviral E2A protein in a subject. In a preferred embodiment, the method for detecting adenoviral infection in a subject comprises determining SUMOylation of the E2A protein in a sample obtained from a subject. In a preferred embodiment, the method for detecting adenoviral infection in a subject comprises determining E2A SUMO conjugates in a sample obtained from a subject.

Similarly, the present invention also provides a method of detecting an adenovirus infection in a subject comprising detecting Sp100A levels and/or its interaction with E2A. The inventors surprisingly found a PML independent interaction between Sp100A and E2A (Fig. 11A, right panel, lane 4). Further, the levels of SP100A protein levels are regulated by E2A SUMOylation as shown in Fig. 11A (left panel, lane 6). Therefore, the inventors have determined a novel role for the viral E2A factor involving Sp100A protein to promote efficient HAdV infection. Therefore, one aspect of the invention is directed to a diagnostic method, where the method comprises determining interaction of E2A and Sp100A and/or levels of Sp100A protein in a subject or portion thereof, wherein increased interaction and/or levels, respectively, indicate replicating adenoviral infection. In particular, provided is a method of detecting an adenovirus infection in a subject, comprising detecting interaction of E2A and Sp100A and/or levels of Sp100A protein in a sample obtained from the subject. Also provided is an ATO therapy, wherein arsenic trioxide is used in a method for the treatment, prevention and/or inhibition of adenovirus infection by reducing, preventing and/or inhibiting adenoviral replication by preventing interaction E2A with Sp100A and/or increased levels of Sp100A in a subject. Rapid and accurate diagnosis of viral infections remains a major hurdle, especially in the low and middle income countries. Currently, the diagnosis of adenoviruses relies on the detection of either the virus antigen or its genome. Although highly sensitive, the reverse transcription-polymerase chain reaction (RT-PCR) is costly and is not affordable for conducting surveillance studies, especially in poor countries. Furthermore, even if a person has adenovirus infection as detected by the viral genome, it does not necessarily mean it is causing the person's particular illness. Some people, especially those who have weakened immune systems, can shed the virus for weeks or longer and not have symptoms. The finding of the present invention that E2A SUMOylation is required for adenoviral replication and the interaction of E2A with Sp100A protein has provided an opportunity for early detection methods of adenoviral infection. This method is feasible and cost effective and also indicates the presence of the virus in an active state and thereby allows the clinicians to attribute a specific disease to the adenovirus infection in a subject.

Accordingly, the present invention provides the use of SUMOylated E2A protein, or E2A SUMO conjugates, as a marker for detecting an adenovirus infection in a subject. In another aspect of the invention, provided is the use of adenoviral Sp100A protein, or E2A Sp100A conjugates, as a marker for detecting an adenovirus infection in a subject. The term "marker" as used herein may be considered to refer to a molecule that is associated quantitatively or qualitatively with the presence of a biological phenomenon, in the present case in particular adenoviral infection. Examples of "markers" may include a polynucleotide, such as a gene or gene fragment, RNA or RNA fragment; or a gene product, including a polypeptide such as a peptide, oligopeptide, polypeptide, protein, or protein fragment; or any related metabolites, by products, or any other identifying molecules, such as antibodies or antibody fragments, whether related directly or indirectly to a mechanism underlying the phenomenon. The markers disclosed herein may include the nucleotide sequences (e.g., GenBank sequences), in particular the full-length sequences, any coding sequences, any fragments, or any complements thereof, and any measurable marker thereof as defined above. "Polypeptide", as used herein, refers to an oligopeptide, peptide, or protein sequence, or fragment thereof, and to naturally occurring, recombinant, synthetic, or semi-synthetic molecules. Where "polypeptide" is recited herein to refer to an amino acid sequence of a naturally occurring protein molecule, "polypeptide" and like terms, are not meant to limit the amino acid sequence to the complete, native amino acid sequence for the full-length molecule. It will be understood that each reference to a "polypeptide" or like term, herein, will include the full-length sequence, as well as any fragments, derivatives, or variants thereof.

The present invention also provides the use of SUMOylated adenoviral E2A protein, or E2A SUMO conjugates or moieties, in a method for detecting an adenovirus infection in a subject. The present invention further provides the use of adenoviral Sp100A protein, or the interaction between adenoviral Sp100A protein and E2A protein, in a method for detecting an adenovirus infection in a subject.

Further provided herein is a diagnostic assay or kit for detecting an adenovirus infection in a subject, comprising means for detecting SUMOylation of E2A protein, preferably means for detecting E2A SUMO conjugates or moieties, in a sample obtained from the subject. Further provided herein is a diagnostic assay or kit for detecting an adenovirus infection in a subject, comprising means for detecting adenoviral Sp100A protein, or detecting the interaction between adenoviral Sp100A protein and E2A protein, in a sample obtained from the subject.

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining E2A SUMO conjugates in the context of a biological sample (e.g., blood, cells, tissue) to thereby determine whether an individual is afflicted with a disease or disorder, or is at risk of developing a disorder, associated with aberrant E2A SUMO conjugates. The invention also provides for prognostic (or predictive) assays for determining whether an individual is at risk of developing a disorder associated with levels of E2A SUMO conjugates. Such assays can be used for prognostic or predictive purpose to thereby prophylactically treat an individual prior to the onset of a disorder characterized by or associated with E2A SUMO conjugates. Another aspect of the invention pertains to monitoring the influence of agents (e.g., drugs, compounds) on the levels of E2A SUMO conjugates in clinical trials.

An exemplary method for detecting the presence or absence of E2A SUMO moieties in a biological sample involves obtaining a biological sample obtained from a test subject and contacting the biological sample with a compound or an agent capable of detecting E2A SUMO moieties such that the presence of E2A SUMO moieties is detected in the biological sample. Suitable probes for use in the diagnostic assays of the invention are described herein. A preferred agent for detecting E2A SUMO moieties is an antibody capable of binding to E2A SUMO moieties, preferably an antibody with a detectable label. Antibodies can be polyclonal, or more preferably, monoclonal. An intact antibody, or a fragment thereof (*e.g*., Fab or F(ab')₂) can be used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

Another aspect of the present invention relates to diagnostic assays for determining adenoviral Sp100A protein, or the interaction between adenoviral Sp100A protein and E2A protein, in the context of a biological sample (e.g., blood, cells, tissue) to thereby determine whether an individual is afflicted with a disease or disorder, or is at risk of developing a disorder, associated with aberrant or increased Sp100A levels. The invention also provides for prognostic (or predictive) assays for determining whether an individual is at risk of developing a disorder associated with aberrant levels of adenoviral Sp100A levels. Such assays can be used for prognostic or predictive purpose to thereby prophylactically treat an individual prior to the onset of a disorder characterized by or associated with aberrant Sp100A levels, or with the interaction between adenoviral Sp100A protein and E2A protein. Another aspect of the invention pertains to monitoring the influence of agents (e.g., drugs, compounds) on the levels of Sp100A protein, or on interaction between adenoviral Sp100A protein and E2A protein in clinical trials.

The term "biological sample" is intended to include, but not limited to, biopsy, tissue sample, cells and biological fluids obtained or isolated from a subject. The diagnostic and detection methods of the invention can be used to detect E2A SUMO conjugates, or Sp100A protein, or the interaction between adenoviral Sp100A protein and E2A protein, in a biological sample *in vitro* as well as *in vivo.* For example, *in vitro* techniques for detection of E2A SUMO conjugates, or the interaction between adenoviral Sp100A protein and E2A protein, may include enzyme linked immunosorbent assays (ELISAs), Western blot, immunoprecipitation and immunofluorescence. Furthermore, corresponding *in vivo* techniques for detection may include introducing into a subject a labeled antibody. For example, the antibody can be labeled with a radioactive marker whose presence, and location in a subject can be detected by standard imaging techniques. In one embodiment, the diagnostic and detection methods may further involve obtaining a control biological sample from a control subject, contacting the control sample with a compound or agent capable of detecting E2A SUMO conjugates (or Sp100A protein, or the interaction between adenoviral Sp100A protein and E2A protein), such that the presence of E2A SUMO conjugates (or Sp100A protein, or the interaction between adenoviral Sp100A protein and E2A protein) is detected in the biological sample, and comparing the presence of E2A SUMO conjugates (or Sp100A protein, or the interaction between adenoviral Sp100A protein and E2A protein) in the control sample with the presence of E2A SUMO conjugates (or Sp100A protein, or the interaction between adenoviral Sp100A protein and E2A protein) in the test sample.

The invention also encompasses kits for detecting the presence of E2A SUMO conjugates (or Sp100A protein, or the interaction between adenoviral Sp100A protein and E2A protein) in a biological sample. For example, the kit can comprise a labeled compound or agent capable of detecting E2A SUMO conjugates (or Sp100A protein, or the interaction between adenoviral Sp100A protein and E2A protein) in a biological sample; means for determining the amount of E2A SUMO conjugates (or Sp100A protein, or the interaction between adenoviral Sp100A protein and E2A protein) in the sample; and means for comparing the amount of E2A SUMO conjugates (or Sp100A protein, or the interaction between adenoviral Sp100A protein and E2A protein) in the sample with a standard. The compound or agent can be packaged in a suitable container. The kit can further comprise instructions for using the kit to detect E2A SUMO conjugates (or Sp100A protein, or the interaction between adenoviral Sp100A protein and E2A protein).

### METHODS

**Cell culture and cell lines.** H1299 (ATCC, No. CRL-5803), HEK293 cells (ECACC European Collection of Authenticated Cell Cultures; Sigma Aldrich, No. 85120602-1VL) and A549 cells (ATCC, No. CCL-185) were grown in Dulbecco's modified Eagle's medium supplemented with 5% fetal calf serum, 100U of penicillin, 100µg of streptomycin per ml in a 5% CO₂ atmosphere at 37 °C. HepG2-NTCP-K7 cells were maintained in Dulbecco's modified Eagle's medium supplemented with 10% fetal bovine serum (FBS), 2mM L-glutamine, 100U of penicillin, 100µg of streptomycin per ml, 1mM sodium pyruvate and non-essential amino acids (Thermo-Fisher Scientific, Waltham, MA, USA) in a 5% CO₂ atmosphere at 37 °C.

HepaRG (66; Thermo Scientific), HepaRG cells stably expressing His/HA/SUMO-2, HepaRG shPML cells (67,68), H1299 (69; ATCC CRL-5803), HEK-293 (Sigma-Aldrich Inc), 2E2 and HeLa cells stably expressing 6His- SUMO-2 were grown in DMEM medium supplemented with 10% FCS, 100 U of penicillin and 100 pg of streptomycin per ml at 37°C in atmosphere with 5% CO₂. For propagation of HepaRG cells media was additionally supplemented with 5 pg/ml of bovine insulin and 0.5 µM of hydrocortisone. HeLa/HepaRG 6His-SUMO/HepaRG shPML cell lines were maintained under 2 µM puromycin selection. 2E2 cells were maintained under 90 pg/pl hygromycin B and 250 pg/pl of G-418. Helper function was induced by 1 pg/ml of doxycycline in the described media. All cell lines are frequently tested for mycoplasma contamination.

**ATO treatment.** ATO was purchased from Sigma Aldrich (St. Louis, MO) and dissolved in 1M NaOH to a stock concentration of 100mM and diluted to 1mM in PBS. For treatment of infected cells, the inoculum was removed, and the medium was exchanged to medium containing ATO in the depicted concentrations.

**Plasmids and transient transfection.** pcDNA-His6-SUMO2 was used for transient transfections, subconfluent H1299 cells were transfected with a mixture of linear polyethylenimine (PEI; 25 kDa) and the respective DNA.

Mutations were introduced into the E2A gene by site-directed mutagenesis using oligonucleotides (Table 1). pCMX3b-Flag E2A (pFlag-E2A-wt) and pCMX3b-Flag E2A (pFlag-E2A) variants were used to transfect HepaRG SUMO-2 cells for Ni-NTA analysis. pCMX3bE2A (pE2A-wt) and pCMX3b-E2AK94/132/202R (pE2A-SCM) as well as pGFP- E2A (pGFP-E2A-wt) and pGFP-E2AK94/132/202R (pGFP-E2A-SCM) cloned into an pEGFP-C1 vector (KpnI and BamHI) were used for transfections for immunoprecipitation analyses in H1299 cells and Ni-NTA analyses in H1299 cells. N-terminally YFP-tagged human Sp100 isoform A construct was generated using specific oligonucleotides (Table 1). N-terminal Flag tagged construct pFlag- Sp100A was used as template. Sp100A was inserted into pEYFP vector using restriction enzymes HindIII and Smal. For yeast experiments, cDNA of selected ORFs (UBE2E1, UBC9, SUM01, PIAS4, OTUB1, SUM02 and full length adenoviral E2A) were amplified via PCR and cloned into pGAD-C1 and pGBD-C1 plasmids using restriction enzymes EcoRI and BamHI. For transient transfections, a mixture of DNA and 25 kDa linear polyethylenimine (Polysciences) was applied to subconfluent cells.

**Cytotoxicity and cell viability assays.** Cell viability in response to infection and treatment with ATO was determined 24h to 48h post infection (p.i.) treatment using the Promega (Madison, WI) CellTiter-Blue Cell Viability Assay system according to the manufacturer's manual. Fluorescence values were detected using a Tecan (Männedorf, Switzerland) Infinite 200M plate reader.

**Viruses.** H5*pg*4100 served as the wild-type HAdV-C5 parental virus (HAdV wt). In addition, a replication competent HAdV-C5 delta E3 virus, encoding a CMV promoter driven eGFP expression cassette, as well as an eGFP expressing HAdV-C5 based first generation adenoviral vector were generated and used in this study. For this, infected cells were harvested after 48h p.i. and lysed by three times of freeze and thaw and reinfected into HEK293 cells. Virus growth was determined by immunofluorescence staining of the adenoviral DNA binding protein E2A.

HAdV E2A SCM mutant virus was also generated and used in the experiments (cf. Fig. 5B):
**K94 Mutant** (SEQ ID NO: 5 showing the region around position K94)
**K132 Mutant** (SEQ ID NO: 6 showing the region around position K132)
**K202 Mutant** (SEQ ID NO: 7 showing the region around position K202)
**K94/K132 Mutant** (SEQ ID NO: 8 and 9 showing the regions around position K94 and K132, respectively)
**K94/132/202 Mutant** (SEQ ID NO: 10, 11 and 12 showing the regions around position K94, K132, and K202, respectively)

This virus carries point mutations in the E2A gene leading to exchange of amino acids at positions 94, 132 and 202 from lysine to arginine, disrupting the SCM in E2A. Viruses were propagated in H1299 cells and titrated in 2E2 cells with an inducible E2A helper function. Virus yield was determined by quantitative HAdV capsid immunofluorescence staining in 2E2 cells. Viral DNA replication was monitored by quantitative PCR using Hexon-specific primers (Table 2). Cells were lysed with RIPA buffer and total DNA was isolated by proteinase K digestion. Samples were diluted 1:100 in nucleic acid-free water (Promega), and 2 pi per sample was mixed with 12.5 µl of Power SYBR Green PCR MasterMix (Roche) and 10 µM concentration of appropriate primer pairs in total volume of 24 µl. Concentration of viral DNA was calculated based on standard curve obtained with quantitative PCR analysis for known concentrations of HAdV bacmid DNA.

**Tecan Measurement.** Sub-confluent H1299 cells in 96-well plates were infected with either wt HAdV-C5, a replication competent HAdV-C5 deltaE3 virus, encoding a CMV promoter driven eGFP expression cassette, or an eGFP expressing HAdV-C5 based first generation adenoviral vector. 2 h p.i., the inoculum was removed and the cells were treated with serial dilutions of ATO in the respective medium. For GFP expressing viruses, the GFP fluorescence was measured 24h, as well as 48 h p.i. using a Tecan Infinite 200M plate reader using an excitation and emission wavelength of 488 and 520 nm. For HAdV-C5 wt, E2A and Capsid proteins were stained by indirect immuno-fluorescence and fluorescence intensity was measured using a Tecan Infinite 200M plate reader using an excitation and emission wavelength of 488 and 520 nm for Alexa 488 (E2A) and 640 and 670 nm for Alexa 647 (Capsid), respectively.

**Determination of viral infection using the IncuCyte S3 Live-Cell Analysis System.** Sub-confluent A549 cells in 96-well plates were infected with a replication competent HAdV-C5 delta E3 virus, encoding a CMV promoter driven eGFP expression cassette. 2h p.i., the inoculum was removed and the cells were treated with serial dilutions of ATO in the respective medium. GFP expression, as well as cell growth were monitored at four positions per well for 48h with a 1h increment using the Sartorius (Göttingen, Germany) IncuCyte S3 Live-Cell Analysis System.

**Yeast two-hybrid assay.** Competent S. cerevisiae were prepared as previously described (74). Yeast two-hybrid expression plasmids pGAD-C1 or pGBD-C1 were used to fuse the GAL4 transcription factor activation domain (AD) or binding domain (BD) to the proteins of interest. AD and BD plasmids contained LEU and TRP as makers, respectively. Co-transformation of these plasmids in PJ69-7A cells was performed following a standard protocol (74) and plated on -LEU-TRP selection media (+HIS) to monitor successful transformation and spotted on -HIS-LEU-TRP selection media to monitor protein-protein-interaction.

**Antibodies and protein analysis.** Primary antibodies specific for adenoviral proteins included E1B-55K mouse mAb 2A6, E4orf6 mouse mAb RSA3, E4orf3 mouse mAb 6A-11, E2A-72K mouse mAb B6-8, E1A mouse mAb M73, and HAdV-5 rabbit polyclonal serum L133. Primary antibodies specific for cellular proteins included monoclonal mouse Ab against the 6xHis epitope (631213; Clontech), polyclonal rabbit Ab raised against the PML protein (NB100-59787; Novus Biologicals), Sp100 rabbit pAb GH3, Mre11 rabbit pAB pNB 100-142 (Novus Biologicals, Inc.), and β-actin mouse mAb AC-15 (Sigma-Aldrich, Inc.).Secondary antibodies conjugated to horseradish peroxidase (HRP) for detection of proteins by immunoblotting were anti-rabbit IgG, anti-mouse IgG, anti-mouse light chain IgG and anti-rat IgG (Jackson/Dianova). All protein extracts were prepared in RIPA lysis buffer as described recently. To detect SUMO2 PTM of E2A, cells were transfected with pcDNA-His6-SUMO2 constructs, cell harvesting was performed on 4°C and denaturating purification was performed. Proteins were separated by SDS-PAGE, transferred to nitrocellulose blotting membranes (0.45µm) and visualized by immunoblotting. Autoradiograms were scanned and cropped using Adobe Photoshop CS5 and figures were prepared using Adobe Illustrator CS5 software. Fiji was used for quantitative analysis and amount comparison of western blots.

**HAdV RNA and DNA synthesis.** Total RNA was isolated with Trizol reagent (Invitrogen) as described by the manufacturer. The amount of total RNA was measured, and one microgram of RNA was reverse transcribed using the Reverse Transcription Kit from Promega including anchored-oligo (dT)15 primer specific to the poly(A)+RNA. Quantitative RT-PCR was performed in a LightCycler 480 (Roche) using 4µl of 1/10 diluted cDNA, 10pmol/µl of the corresponding oligonucleotide primers and 5µl of SYBR Green Mastermix (Roche) per sample. The following PCR conditions were used: 10 min at 95°C and 40 cycles of 30s at 95°C, 30s at 62°C and 30s at 72°C. The viral mRNA levels obtained from triplicate reactions were calculated in relation to levels of the cellular 18S mRNA. For the analysis of viral DNA, protein lysates were digested with Proteinase K (PK) for 1h at 55°C and boiled afterwards for 10min at 95°C to inactivate the PK. Samples were then subjected to quantitative PCR analysis in a LightCycler 480 (Roche) using 4µl of 1/200 diluted DNA, 10pmol/µl of the corresponding oligonucleotide primers and 5µl of SYBR Green Mastermix (Roche) per sample. The following PCR conditions were used: 10min at 95°C and 40 cycles of 30s at 95°C, 30s at 62°C and 30s at 72°C. The viral DNA levels obtained from triplicate reactions were calculated in relation to levels of the cellular gapdh coding region. Primers are listed in Table 2.

**Viral capsid formation.** Cells for determination of viral capsid formation were resuspended in a low stringent lysis buffer (50mM Tris-Cl (pH 8.0), 100 mM NaCl, 1mM EDTA, and 1% NP-40) and incubated for 10min on ice. Samples were centrifuged at 12.000 *xg*/4°C. Samples were mixed with 6x loading buffer (50% glycerol and 0.1% bromophenolblue) and subjected to agarose gel electrophoresis. Proteins were transferred to nitrocellulose blotting membranes (0.2µm) by capillary transfer using 10x saline sodium citrate (SSC) buffer and visualized by immunoblotting. Autoradiograms were scanned and cropped using Adobe Photoshop CS5 and figures were prepared using Adobe Illustrator CS5 software. Fiji was used for quantitative analysis and amount comparison of western blots.

**Chromatin immunoprecipitation (ChIP) assay.** HepaRG cells were transfected with pFlag E2A-wt or pFlag E2A SCM constructs and after 4h infected with corresponding HAdV. The cells were cross- linked with 1% methanol-free formaldehyde (ThermoFisher Scientific) for 10 min at room temperature, 24 h after infection. Glycine was added to a final concentration of 125 mM to stop the cross-linking reaction, and the samples were incubated for another 5 min at room temperature. Cells were washed twice with ice-cold PBS, harvested, and lysed in SDS buffer (50 mM Tris-HCl [pH 8.1], 10 mM EDTA, 1% SDS). Samples were incubated on ice for 10 min, sonicated for 15 min in a Diagenode Bioruptor Pico (30-s on-off intervals) and cleared by centrifugation two times for 10 min at 16,000 χ g and 4°C. For ChIP, sheared chromatin from 1.5 χ 106 cells in 50 µl (Flag/E2A-ChIP) was combined with 9 volumes of ChIP dilution buffer (16.7 mM Tris-HCl [pH 8.1], 167 mM NaCl, 1.2 mM EDTA, 1.1% Triton X-100, 0.01% SDS) and subjected to immunoprecipitation for 16 h at 4°C with gentle rotation using 5 µg mouse anti-Flag M2 antibody (Sigma-Aldrich, F3165), or 5 µg normal mouse IgG (Sigma-Aldrich, 15381). Samples were centrifuged for 10 min at 20,000 χ g and 4°C to remove any precipitated material, and the supernatants were combined with 20 pi Magna ChIP protein G (E2A/Flag-ChIP) magnetic beads (Millipore) and incubated for 2 h at 4°C with gentle rotation. Immune complexes were washed consecutively with 1 ml each of low-salt buffer (20 mM Tris-HCl [pH 8.1], 150 mM NaCl, 2 mM EDTA, 1% Triton X-100, 0.1% SDS), high-salt buffer (20 mM Tris [pH 8.1], 0.5 M NaCl, 2 mM EDTA, 1% Triton X-100, 0.1% SDS), and lithium chloride buffer (10 mM Tris [pH 8.1], 0.25 M LiCl, 1 mM EDTA, 1% IGEPAL CA-630, 1% deoxycholic acid), and twice with TE (10 mM Tris-HCl [pH 8.0], 1 mM EDTA) buffer. Elution of the chromatin-antibody complexes was carried out by incubation with 150 pi freshly prepared elution buffer (100 mM NaHCO3, 1% SDS) containing 1.5 pi proteinase K (Roche) at 62°C for 2 h, followed by a 10 min incubation step at 95°C. DNA was purified using the MinElute PCR Purification Kit from Qiagen according to manufacturer's instructions. DNA was eluted twice with 20 µl buffer EB, and 2 µl of the DNA solution was used as template DNA for qPCR with primers listed in Table 2.

**Table 1 - Cloning Primers used**

| **Sequence IDs** | **Name** | **Sequence (5'->3')** |
|---|---|---|
| **SEQ ID NO: 13** | Sp100A fwd | ATAAAGCTTATATGGCAGGTGGGGGCGGC |
| **SEQ ID NO: 14** | Sp100A rev | TATCCCGGGCATCTTCTTTACCTGACCCTCTTC |
| **SEQ ID NO: 15** | E2A K94R fwd | GAAGCGCCCTTCTCCCAGGCCCGAGCGCCCG |
| **SEQ ID NO: 16** | E2A K94R rev | CGGGCGCTCGGGCCTGGGAGAAGGGCGCTTC |
| **SEQ ID NO: 17** | E2A K202R fwd | GTG ATAACGATCTAAGGGCGAACTTCAAACTAC |
| **SEQ ID NO: 18** | E2A K202R rev | GTAGTTTGAAGTTCGCCCTTAGATCGTTATCCAC |
| **SEQ ID NO: 19** | E2A K132R fwd | GCTAATCAAGCATGGCAGAGGAGGTAAGCGCACAG |
| **SEQ ID NO: 20** | E2A K132R rev | CTGTGCGCTTACCTCCTCTGCCATGCTTGATTAGC |

**Table 2 - qPCR primers used**

| **Sequence IDs** | **Name** | **Sequence (5'->3')** | **Target** |
|---|---|---|---|
| **SEQ ID NO: 21** | HAdV P1 fwd | CTTGCATGGCGTGTTAAATG | HAdV 1635-1654 |
| **SEQ ID NO: 22** | HAdV P1 rev | GCCTCCATGAGGTCAGATGT | HAdV 1702-1721 |
| **SEQ ID NO: 23** | HAdV P3 fwd | GGTGCACTTGGGAAATTTGT | HAdV 4508-4527 |
| **SEQ ID NO: 24** | HAdV P3 rev | TATGGACGAATGCATGGAAA | HAdV 4588-4607 |
| **SEQ ID NO: 25** | HAdV P7 fwd | TGACGAGGACGATGAGTACG | HAdV 12255-12274 |
| **SEQ ID NO: 26** | HAdV P7 rev | GTTGCGTCTTGCATCATCTG | HAdV 12315-12334 |
| **SEQ ID NO: 27** | HAdV P11 fwd | CTGTGGGTGATAACCGTGTG | HAdV 19101-19120 |
| **SEQ ID NO: 28** | HAdV P11 rev | TAAAAGTAGGGCCCCTGTCC | HAdV 19162-19181 |
| **SEQ ID NO: 29** | HAdV P16 fwd | AACGCCATAGTTGCTTGCTT | HAdV 26588-26607 |
| **SEQ ID NO: 30** | HAdV P16 rev | ACGCCGTGATGGTAGAGAAG | HAdV 26648-26667 |
| **SEQ ID NO: 31** | GAPDH fwd | CATCCTGGGCTACACTGA | |
| **SEQ ID NO: 32** | GAPDH fwd | TTGACAAAGTGGTCGTTG | |
| **SEQ ID NO: 33** | EIA fwd | GTGCCCCATTAACCAGTTG | |
| **SEQ ID NO: 34** | E1A rev | GGCGTTTACAGCTCAAGTCC | |
| **SEQ ID NO: 35** | Hexon fwd | CGCTGGACATGACTTTTG | |
| **SEQ ID NO: 36** | Hexon rev | AGGAACGGTGTGCGCAGGTA | |
| **SEQ ID NO: 37** | 18S fwd | CGGCTACCACATCCAAGGAA | |
| **SEQ ID NO: 38** | 18S rev | GCTGGAATTACCGCGGCT | |

### EXAMPLES

Embodiments of inventive methods are illustrated in the following examples. These examples are provided for illustrative purposes and are not considered limitations on the scope of the inventive methods.

### Example 1- ATO antagonizes productive adenovirus infection cycles

Human carcinoma cells with HAdV wild type virus were treated with different concentrations of ATO. ATO concentrations between 0 and 2.0µM were used. These initial studies determined a dose-dependent anti-HAdV activity with 40% reduction of early viral DNA binding protein E2A expression at 1µM, and almost 80% inhibition of late capsid protein expression at 2.0µM ATO (Fig. 1A). Virus progeny production upon ATO treatment was also observed. Treatment with 1µM ATO was associated with 80% reduction of virus yield, while 2µM ATO abolished virus particle synthesis compared to non-treated and infected control cells (Fig. 1B).

### Example 2- ATO blocks efficient HAdV gene expression.

Human carcinoma cells with HAdV wild type virus were treated with different concentrations of ATO. ATO concentrations (0-10 µM) were used.

### Protein Expression

E2A (24/48h p.i.)/ capsid (48h p.i.) protein concentrations were measured (p.i.: post infection). 8µM ATO showed the ability to completely block HAdV E2A protein expression, however less than 50% E2A expression compared to the untreated control were already observed with 4µM ATO after 24h p.i. (IC50 1.910µM; Fig. 2A). At 48h p.i., 4µM ATO reduced E2A expression to 60% and was not detectable with 8µM ATO (IC50 4.062µM). For capsid expression after 48h p.i., a 40% reduction with 2µM ATO and complete loss of capsid detection with 4µM ATO (IC50: 2.067µM) was observed. The cellular cytotoxicity of ATO in these assays were analyzed simultaneously and showed no clear effect (Fig. 2A, lower panel). Using HAdV wt infection, it is shown that ATO efficiently reduces the amount of cells infected and expressing early E2A and late capsid protein (Fig. 2A).

### GFP Expression Cassette

To further validate significant reduction of HAdV gene expression, a replication competent HAdV was generated with an inserted GFP expression cassette in the viral genome. Successful GFP transcription from an inserted CMV promoter was measured and quantified to assess HAdV productive infection. Highly efficient inhibition of HAdV infection with ATO after 24 and 48h p.i. (Fig. 2B) was observed. Data indicates 50% reduction of HAdV infection under treatment with 2µM ATO after 24h p.i. (IC50 1.610µM), which was even stronger after 48 h p.i. (IC50 1.177µM). Again, the cellular cytotoxicity of ATO in these assays was analyzed simultaneously and showed no clear effect (Fig. 2B, lower panel).

### Viral transcript levels

To clarify ATO capacity to block viral gene expression without viral spread occurring, an assay to measure viral transcript levels was carried out using a non-replicating eGFP expressing HAdV-C5 based first generation adenoviral vector. Different ATO concentrations (0-8µM) were used and GFP protein concentrations were measured. Less than 50% GFP expression compared to the untreated control was only observed with 8µM ATO after 24h (IC50 6.485µM) and 48h p.i. (IC50 10.240µM; Fig. 2C). The cellular cytotoxicity of ATO in these assays were analyzed simultaneously and showed no clear effect (Fig. 2C, lower panel).

Using replication incompetent HAdV infection, it was shown that ATO affects either the entry process or the transcription of the GFP cassette in the viral genome.

### Real-time kinetic data

To further validate HAdV block by ATO, real-time kinetic data was determined with a dilution of different ATO concentrations after HAdV-GFP wt infection and monitored every second hour up to 60h p.i. with an IncuCyte® automated live-cell imaging system. Fluorescence images were taken every 2h for 60h in a 37°C CO₂ incubator. ATO efficiently represses GFP expression over time with a four-fold reduction at 60h p.i. (Fig. 2D).

### Example 3 - ATO reduces HAdV DNA and RNA synthesis.

### DNA Replication

The effect of ATO upon efficient HAdV DNA replication using quantitative real-time PCR (qPCR) was examined. Cells were infected with HAdV and incubated for 24h or 48h. HAdV DNA was extracted and quantitative PCR was performed to quantify the synthesis of new HAdV DNA as a measure of DNA replication efficiency. The presence of ATO shows a significant effect in blocking HAdV DNA replication treated with 1µM (60% reduction) and 2µM (80% reduction) of the drug (Fig. 3A). Quantification of GAPDH DNA was used as a control and did not show a significant effect.

### mRNA transcription

To assay the inhibition of HAdV mRNA transcription by ATO, cells were infected in the presence of the compound (0-2µM) for 2h p.i. and harvested 48h later. After infection, mRNA copy number of E1A (early gene) and Hexon (late gene) were quantified using quantitative RT-PCR. Results showed significant differences in total E1A/Hexon mRNA levels (Fig. 3B). For both viral transcripts, 40% reduction at 1µM ATO was observed compared with the control treated with PBS. 80% (E1A) and 60% (Hexon) inhibition of viral mRNA expression was detected at 2µM, without significant impact of the compound on host 18S mRNA. Thus, ATO directly affects early and late viral mRNA production.

### Example 4 - ATO significantly reduces HAdV protein synthesis.

### Protein Expression

Western blot analysis was used for viral protein expression under 1 and 2µM ATO treatment, viral early and late protein synthesis was significantly impaired (Fig. 3C). Consistent with the delay in viral mRNA synthesis under ATO treatment, a decrease in subsequent expression of the early viral protein E2A and a decrease in late capsid and capsid associated proteins (Fig. 3C) was also detected. Under ATO treatment, a decrease in E1B-55K and E4orf6 viral proteins (Fig. 3C) was detected. These two viral factors are viral components of an E3 ubiquitin ligase complex, which is essential for efficient virus replication due to proteasomal degradation of host substrates. In line with these facts, an insufficient proteasomal degradation in infected cells treated with ATO compared to untreated cells (data not shown) was observed, resulting in a more efficient inhibition of antiviral components of the DNA repair pathway.

### Example 5 - ATO interferes with efficient virus particle assembly.

The impact of ATO treatment on HAdV assembly was monitored. A low stringent lysis of infected cells was performed in the presence of ATO prior to separation of native lysates with agarose gel electrophoresis and immunoblotting using capsid antibody. Proper capsid formation without ATO treatment in infected cells was detected (Fig. 3D, left panel). Native gel assay showed that ATO presence significantly reduced the signal obtained after capsid staining, however also capsid protein expression was reduced (Fig 3D, right panel). Normalization to capsid input strengthened the observation that ATO blocks HAdV capsid assembly (Fig. 3D, right panel).

### Example 6 - ATO alters HAdV RC localization.

HAdV localize their genomes juxtaposed to PML-NBs for the formation of replication and transcription domains (RCs; replication centers), i.e. in close proximity to PML-NBs. ATO treatment significantly decreases the number of PML-NBs in non-infected cells (Fig. 4A). Intriguingly, after ATO treatment in HAdV infected cells, we observed a significant reduction of RC in number (Fig. 4B).

### Example 7 - HAdV E2A is a novel target of the host SUMO conjugation machinery.

HAdV utilize host SUMOylation processes to enhance viral replication and evade cellular immune responses. Potential interactors by yeast two-hybrid experiments (Y2H) were searched for and revealed, in at least one Y2H combination setting, a novel direct interaction between E2A and the SUMO conjugating enzyme Ubc9, but not with the SUMO E3 ligase PIAS4 (Fig. 5A). To ensure specificity, interaction of Ubc9 with various elements of the SUMO conjugation machinery was confirmed, such as PIAS4, SUM01 and SUM02.

### SUMO GPS Software

Based on these findings, *SUMO GPS software* was used to determine putative SCMs *(SUMO conjugation motifs)* within the E2A protein. Highly scored E2A SCMs were identified and used to exchange the respective lysines for arginines by site-directed mutagenesis (Fig. 5B). E2A wt and E2A SCM variants with Flag expressing epitopes were then transfected into human cells stably expressing His-SUMO-2 (Fig. 5C).

### Purification and SDS-PAGE

Ni-NTA purified His-SUMO-2 conjugates and total-cell lysates were subjected to Western blot analyses, which revealed an E2A signal at 72 kDa and higher migrating protein bands (Fig. 5C, right panel, lane 2). Since covalent SUMO attachment to a substrate protein increases the molecular weight by 20 to 40 kDa, these higher migrating bands correspond to SUMOylated moieties of the adenoviral protein. Less SUMO binding to E2A K94R, K132R and K202R was detected than to the wt protein (pE2A-wt; Fig. 5C, right panel, lanes 2-5). pE2A-K94/132R, and triple SCM mutant pE2A-K94/132/202R (pE2A-SCM) generated to efficiently reduce covalent SUMOylation of the viral factor, showed even less SUMO binding, in particular E2A SUMO conjugation was drastically diminished when all three putative SCM motifs were disrupted (Fig. 5C, lane 7).

### Immunoprecipitation

To further confirm that Ubc9 interacts with E2A in mammalian systems and thereby serves as a SUMO conjugating enzyme for E2A, cells were transfected with pE2A-wt and pE2A-SCM plasmids tagged with GFP-expressing epitopes. Immunoprecipitation analyses of GFP tagged E2A and subsequent staining for endogenous Ubc9 revealed an interaction between E2A and Ubc9. As this binding was absent in cells transfected with the triple SCM mutant pE2A-K94/132/202R (pE2A-SCM; Fig. 5D, right panel, lanes 2 and 3), this variant was included into further investigations on the role of E2A SUMO PTM.

### E2A SUMO PTM

It was tested whether the lysine to arginine exchange in the E2A SCM simultaneously affects E2A ubiquitin PTM or only SUMOylation. Therefore, E2A wt and E2A SCM versions were transfected into human cells in combination with His-Ubiquitin (Ubi) (Fig. 5E). Ni-NTA purified His-Ubi conjugates and total-cell lysates were subjected to Western blot analyses and showed a similar Ubiquitin PTM above 72 kDa, when E2A was detected with specific antibody after His purification (Fig. 5E, right panel, lanes 5 and 6).

### Example 8 - Generation and validation of an E2A SCM mutant virus.

### Purification and SDS-PAGE

To elucidate the role of E2A SUMOylation during the course of adenoviral infection, a mutant virus was generated mimicking the mutant pE2A-SCM construct, where lysine residues K94/132/202 are substituted by arginine to reduce SUMOylation of the viral protein. A two-plasmid system was used with a large viral bacmid and a small region-specific plasmid (L4-Box) to insert point mutations into E2A in the 36 kb linear genome of HAdV (Fig. 6A). Analyses of Ni-NTA purified samples from infected HepaRG His/HA SUMO-2 cells revealed that E2A SUMO chains are diminished during infection with E2A SCM mutant virus (HAdV E2A SCM) compared to wt virus (HAdV wt) infection (Fig. 6B, right panel, lanes 5 and 6). E2A SUMOylation in E2A SCM mutant infected cells was reduced by 70 % compared to HAdV wt infection (Fig. 6C). These results concurred with the transfection experiments above (Fig. 5C, D), confirming that E2A is a novel viral target of the cellular SUMO conjugation machinery, and that triple SCM mutation of K94/132/202 efficiently reduced PTM.

### Example 9 - E2A SUMOylation controls PML binding to the viral factor in infected cells.

SUMOylation is involved in regulating the protein/protein interactions of its substrates in cells. As the scaffold protein of PML-NBs, PML preferentially recruits SUMOylated proteins through SUMO/SIM interactions. Thus, it was investigated whether SUMOylation of E2A affects PML binding independently of any other viral component.

### Immunoprecipitation

Immunoprecipitation of cells transfected with E2A encoding plasmid pE2A-wt and pE2A-SCM showed that E2A SUMO conjugation does not influence PML binding without the viral background (Fig. 7A, right panel, lanes 3 and 4; Fig. 7B). To investigate the influence of E2A SUMOylation on E2A/PML binding during HAdV infection, immunoprecipitation of PML from cells infected with HAdV wt, HAdV E2A SCM or HAdV AE4orf3 viruses was performed. Subsequent detection of E2A revealed that E2A interaction with PML during infection depends on E2A SUMO PTM (Fig. 7C, right panel, lanes 3 and 4 and 7D). Compared to the wt infection, E2A/PML binding decreased by 78.2% when E2A SUMOylation was reduced by the triple SCM mutation (Fig. 7C and 7D). Additional investigation of a virus mutant lacking E4orf3 showed no effect of this viral factor on E2A/PML binding (Fig. 7C, right panel, lane 5). In sum, these data suggest that E2A SUMOylation regulates E2A/PML interaction during HAdV infection.

### Example 10 - Effects of E2A SUMOylation on numbers of HAdV RC

Effects of E2A SUMOylation on the number of HAdV RC during infection were investigated. The number of HAdV RCs per infected cell was reduced by 26.9% during HAdV E2A SCM infection compared to HAdV wt infection (Fig. 8). Taken together, it is shown that E2A SUMO PTM represents a potential link to the number of HAdV RCs.

### Example 11 - SUMOylation of E2A is beneficial for HAdV replication.

To investigate, whether E2A SUMOylation affects production of adenoviral progeny, virus growth was determined in HepaRG cells (Fig. 9A). The results pointed to progeny production depending on E2A SUMOylation at all three time points examined post infection. A 62% reduction in viral progeny production 24h p.i. with HAdV E2A SCM virus was detected. Compared to HAdV wt infection, production of viral progeny was reduced by 41% after 48 h, and reached 50% after 72h (Fig. 9A).To examine the influence of SUMO PTM on E2A stability, protein levels of E2A during infection with HAdV wt and HAdV E2A SCM virus were monitored. Even though the data showed that viral progeny production depends on E2A SUMOylation (Fig. 9A). Consistent with the virus yield data, we observed reduced amounts of late protein expression during E2A SCM mutant virus infection compared to HAdV wt, 30, 48 and 72h p.i. (Fig. 9C). Similar results were obtained monitoring viral E1A and Hexon (Fig. 9B) mRNA synthesis in infected cells.

### Example 12 - ATO interferes with E2A SUMOylation during HAdV infection.

Due to its essential role in HAdV replication, E2A is termed as marker for HAdV replication centers. Biochemical analysis revealed viral E2A as a novel interaction partner of PML. Host SUMOylation machinery targets E2A, facilitating E2A protein functions and host interactions. E2A SUMOylation status in ATO treated infected cells were investigated. NiNTA pull down assays in infected cells under 2µM ATO were performed (Fig. 10A). Compared to the untreated controls, data indicates that ATO significantly decreases the presence of E2A SUMO moieties in infected cells by 70% compared to untreated cells (Fig. 10B).

### Example 13 - Diagnostic Method to detect Adenoviral infection with E2A SUMOylation

To detect SUMO2 PTM of E2A or E2A SUMO moieties, cells were transfected with pcDNA-His6-SUMO2 constructs, cell harvesting was performed on 4°C and denaturation purification was performed. Proteins were separated by SDS-PAGE, transferred to nitrocellulose blotting membranes (0.45µm) and visualized by immunoblotting. Autoradiograms were scanned and cropped using Adobe Photoshop CS5 and figures were prepared using Adobe Illustrator CS5 software. Fiji was used for quantitative analysis and amount comparison of western blots. The same principle and experimental methods, with or without modifications, can be used to detect E2A SUMO moieties in a biological sample obtained from a subject that may or may not have E2A SUMO moieties thereby confirming the presence or absence of replicating adenovirus. An exemplary method for detecting the presence or absence of E2A SUMO moieties in a biological sample involves obtaining a biological sample from a subject and contacting the biological sample with a compound or an agent capable of detecting E2A SUMO moieties such that the presence of E2A SUMO moieties is detected in the biological sample.

### Example 14- E2A SUMOylation promotes interaction with the HAdV transcriptional activator Sp100A

Sp100A is associated with PML-NBs during wt HAdV infection, independent on the E2A SUMO status. After human cells were transfected with pYFP-Sp100A and superinfected with wt HAdV or E2A SCM virus mutant, immunoprecipitation of YFP and staining for E2A revealed an interaction between Sp100A and E2A SUMO-moieties. (Fig. 11A, right panel, lane 4; Fig. 11C). Reduced amount of E2A protein was detected, when Sp100A interaction was monitored in presence of the SCM mutant variant, confirming an E2A SUMO dependent interaction between the viral factor and the host transcription factor (Fig. 11A, right panel, lane 6; Fig. 11C). We note, that wt HAdV infection increased Sp100A protein levels (Fig. 11A, left panel, lane 4) compared to SCM virus mutant infections (Fig. 11A, left panel, lane 6). To next determine whether PML mediates Sp100A/E2A interaction, PML-depleted cells were transfected with pYFP-Sp100A, and subsequently infected with wt HAdV or E2A SCM virus mutant. Immunoprecipitation of YFP and staining for E2A revealed a PML independent interaction between Sp100A and E2A (Fig. 11C, right panel, lane 4). Taken together, Sp100A/E2A interaction (Fig. 11B, right panel, lane 6; Fig. 11D), and Sp100A protein levels (Fig. 11B, left panel, lane 6) are regulated by E2A SUMOylation also in the absence of PML; this result highlights a novel role for the viral E2A factor regulated by its own SUMO PTM to promote efficient HAdV infection.

### Example 15 - Mechanism of adenoviral replication and ATO treatment response

The mechanism of adenoviral replication, E2A SUMOylation and response to ATO treatment provided is summarized in Fig. 12. Upon infection with adenovirus (Fig. 12A), E2A protein associates with PML and forms E2A SUMO moieties. Upon treatment with arsenic trioxide (Fig. 12B), E2A is de-SUMOylated, which inhibits adenoviral replication. The adenoviral infection is therefore decreased (Fig. 12C).

## Claims

1. Arsenic trioxide (As₂O₃) for use in the treatment or prevention of an adenovirus infection in a subject.

2. Arsenic trioxide for the use according to claim 1, wherein arsenic trioxide is preventing and/or inhibiting recurrent, persistent, and/or *de novo* viral infection.

3. Arsenic trioxide for the use according to claim 1 or 2, wherein arsenic trioxide is preventing and/or inhibiting replication of adenovirus.

4. Arsenic trioxide for the use according to claim 1, wherein arsenic trioxide is reducing the adenoviral load.

5. Arsenic trioxide for the use according to any one of claims 1 to 4, wherein the subject is a subject suffering from, or at risk of contracting, a disease or disorder associated with, or caused by, an adenovirus infection, preferably wherein the use is for lessening the severity of clinical symptoms in the subject suffering from the disease or disorder associated with, or caused by, an adenovirus infection.

6. Arsenic trioxide for the use according to claim 5, wherein the disease or disorder associated with, or caused by, an adenovirus infection is any one of a disease or disorder of the respiratory system, conjunctivitis, keratoconjunctivitis, gastroenteritis, pneumonia, hepatitis, encephalitis, or cystitis.

7. Arsenic trioxide for the use according to any one of claims 1 to 6, wherein the subject is an immunocompromised subject, preferably wherein the immunocompromised subject is a solid-organ transplant recipient, a stem cell transplant recipient, or a subject suffering from cancer and/or an additional chronic infection.

8. Arsenic trioxide for the use according to any one of claims 1 to 7, further comprising treatment of the subject with an anti-adenovirus agent, which is not arsenic trioxide.

9. Arsenic trioxide for the use according to any one of claims 1 to 8, wherein the arsenic trioxide is to be administered at a low dose of 0.15 mg/kg/d or less.

10. A kit comprising a therapeutically effective amount of arsenic trioxide (As₂O₃) and instructions for the administration of arsenic trioxide to a subject suffering from an adenovirus infection, or at risk of being infected with an adenovirus.

11. The kit of claim 10, further comprising an anti-adenovirus agent, which is not arsenic trioxide.

12. A method of detecting an adenovirus infection in a subject, comprising detecting SUMOylation of adenovirus E2A protein, preferably detecting E2A SUMO conjugates, in a sample obtained from the subject.

13. Use of SUMOylated adenovirus E2A protein, or E2A SUMO conjugates, as a marker for detecting an adenovirus infection in a subject.

14. Use of SUMOylated adenovirus E2A protein, or E2A SUMO conjugates, in a method for detecting an adenovirus infection in a subject.

15. A diagnostic assay or kit for detecting an adenovirus infection in a subject, comprising means for detecting SUMOylation of adenovirus E2A protein, preferably means for detecting E2A SUMO conjugates, in a sample obtained from the subject.

16. Arsenic trioxide for the use according to any one of claims 1 to 9, or the kit according to claim 10 or 11, or the method of claim 12, or the use according to claim 13 or 14, or the diagnostic assay or kit according to claim 15, wherein the subject is a mammal, preferably a human.

17. Arsenic trioxide for the use according to any one of claims 1 to 9 and 16, or the kit according to any one of claims 10, 11, and 16, or the method of claim 12 or 16, or the diagnostic assay or kit according to claim 15 or 16, or the use according to any one of claims 13, 14 and 16, wherein the adenovirus is human adenovirus (HAdV).


SEQUENCE LISTING

<110>  Technische Universität München

<120>  New means and methods for therapy and diagnosis of adenoviral
infection

<130>  EP124363-RPpau

<140>  not yet assigned
<141>  herewith

<160>  38

<170>  PatentIn version 3.5

<210>  1
<211>  101
<212>  PRT
<213>  Homo sapiens

<220>
<223>  SUMO-1

<400>  1

Met Ser Asp Gln Glu Ala Lys Pro Ser Thr Glu Asp Leu Gly Asp Lys
1               5                   10                  15

Lys Glu Gly Glu Tyr Ile Lys Leu Lys Val Ile Gly Gln Asp Ser Ser
            20                  25                  30

Glu Ile His Phe Lys Val Lys Met Thr Thr His Leu Lys Lys Leu Lys
        35                  40                  45

Glu Ser Tyr Cys Gln Arg Gln Gly Val Pro Met Asn Ser Leu Arg Phe
    50                  55                  60

Leu Phe Glu Gly Gln Arg Ile Ala Asp Asn His Thr Pro Lys Glu Leu
65                  70                  75                  80

Gly Met Glu Glu Glu Asp Val Ile Glu Val Tyr Gln Glu Gln Thr Gly
                85                  90                  95

Gly His Ser Val Thr
            100

<210>  2
<211>  94
<212>  PRT
<213>  Homo sapiens

<220>
<223>  SUMO-2

<400>  2

Met Ala Asp Glu Lys Pro Lys Glu Gly Val Lys Thr Glu Asn Asn Asp
1               5                   10                  15

His Ile Asn Leu Lys Val Ala Gly Gln Asp Gly Ser Trp Gln Phe Lys
            20                  25                  30

Ile Lys Arg His Thr Pro Leu Ser Lys Leu Met Lys Ala Tyr Cys Glu
        35                  40                  45

Arg Gln Gly Leu Ser Met Arg Gln Ile Arg Phe Arg Phe Asp Gly Gln
    50                  55                  60

Pro Ile Asn Glu Thr Asp Thr Pro Ala Gln Leu Glu Met Glu Asp Glu
65                  70                  75                  80

Asp Thr Ile Asp Val Phe Gln Gln Gln Thr Gly Gly Val Tyr
                85                  90

<210>  3
<211>  102
<212>  PRT
<213>  Homo sapiens

<220>
<223>  SUMO-3

<400>  3

Met Ser Glu Glu Lys Pro Lys Glu Gly Val Lys Thr Glu Asn Asp His
1               5                   10                  15

Ile Asn Leu Lys Val Ala Gly Gln Asp Gly Ser Trp Gln Phe Lys Ile
            20                  25                  30

Lys Arg His Thr Pro Leu Ser Lys Leu Met Lys Ala Tyr Cys Glu Arg
        35                  40                  45

Gln Gly Leu Ser Met Arg Gln Ile Arg Phe Arg Phe Asp Gly Gln Pro
    50                  55                  60

Ile Asn Glu Thr Asp Thr Pro Ala Gln Leu Glu Met Glu Asp Glu Asp
65                  70                  75                  80

Thr Ile Asp Val Phe Gln Gln Gln Thr Gly Gly Val Pro Glu Ser Ser
                85                  90                  95

Leu Ala Gly His Ser Phe
            100

<210>  4
<211>  94
<212>  PRT
<213>  Homo sapiens

<220>
<223>  SUMO-4

<400>  4

Met Ala Asn Glu Lys Pro Thr Glu Glu Val Lys Thr Glu Asn Asn Asn
1               5                   10                  15

His Ile Asn Leu Lys Val Ala Gly Gln Asp Gly Ser Trp Gln Phe Lys
            20                  25                  30

Ile Lys Arg Gln Thr Pro Leu Ser Lys Leu Met Lys Ala Tyr Cys Glu
        35                  40                  45

Pro Arg Gly Leu Ser Met Lys Gln Ile Arg Phe Arg Phe Gly Gly Gln
    50                  55                  60

Pro Ile Ser Gly Thr Asp Lys Pro Ala Gln Leu Glu Met Glu Asp Glu
65                  70                  75                  80

Asp Thr Ile Asp Val Phe Gln Gln Pro Thr Gly Gly Val Tyr
                85                  90


<210>  5
<211>  14
<212>  PRT
<213>  Artificial Sequence

<220>
<223>  Region around K94 mutation

<400>  5

Lys Lys Arg Pro Ser Pro Lys Pro Glu Arg Pro Pro Ser Pro
1               5                   10


<210>  6
<211>  14
<212>  PRT
<213>  Artificial Sequence

<220>
<223>  Region around K132 mutation

<400>  6

Val Leu Ile Lys His Gly Lys Gly Gly Lys Arg Thr Val Arg
1               5                   10


<210>  7

<211>  14
<212>  PRT
<213>  Artificial Sequence

<220>
<223>  Region around K202 mutation

<400>  7

His Val Asp Asn Asp Leu Lys Ala Asn Phe Lys Leu Ile Pro
1               5                   10


<210>  8
<211>  8
<212>  PRT
<213>  Artificial Sequence

<220>
<223>  Region around K94 mutation in K94/K132 Mutant

<400>  8

Pro Ser Pro Lys Pro Glu Arg Pro
1               5


<210>  9
<211>  8
<212>  PRT
<213>  Artificial Sequence

<220>
<223>  Region around K132 mutation in K94/K132 Mutant

<400>  9

Lys His Gly Lys Gly Gly Lys Arg
1               5


<210>  10
<211>  8
<212>  PRT
<213>  Artificial Sequence

<220>
<223>  Region around K94 mutation in K94/132/202 Mutant

<400>  10

Pro Ser Pro Lys Pro Glu Arg Pro
1               5


<210>  11
<211>  8
<212>  PRT
<213>  Artificial Sequence

<220>
<223>  Region around K132 mutation in K94/132/202 Mutant

<400>  11

Lys His Gly Lys Gly Gly Lys Arg
1               5


<210>  12
<211>  8
<212>  PRT
<213>  Artificial Sequence

<220>
<223>  Region around K202 mutation in K94/132/202 Mutant

<400>  12

Asn Asp Leu Lys Ala Asn Phe Lys
1               5


<210>  13
<211>  29
<212>  DNA
<213>  Artificial Sequence

<220>
<223>  primer

<400>  13
ataaagctta tatggcaggt gggggcggc                                         29


<210>  14
<211>  33
<212>  DNA
<213>  Artificial Sequence

<220>
<223>  primer

<400>  14
tatcccgggc atcttcttta cctgaccctc ttc                                    33


<210>  15
<211>  31
<212>  DNA
<213>  Artificial Sequence

<220>
<223>  primer

<400>  15
gaagcgccct tctcccaggc ccgagcgccc g                                      31


<210>  16
<211>  31
<212>  DNA
<213>  Artificial Sequence

<220>
<223>  primer

<400>  16
cgggcgctcg ggcctgggag aagggcgctt c                                    31


<210>  17
<211>  33
<212>  DNA
<213>  Artificial Sequence

<220>
<223>  primer

<400>  17
gtgataacga tctaagggcg aacttcaaac tac                                  33


<210>  18
<211>  34
<212>  DNA
<213>  Artificial Sequence

<220>
<223>  primer

<400>  18
gtagtttgaa gttcgccctt agatcgttat ccac                                 34


<210>  19
<211>  35
<212>  DNA
<213>  Artificial Sequence

<220>
<223>  primer

<400>  19
gctaatcaag catggcagag gaggtaagcg cacag                                35


<210>  20
<211>  35
<212>  DNA
<213>  Artificial Sequence

<220>
<223>  primer

<400>  20
ctgtgcgctt acctcctctg ccatgcttga ttagc                                35


<210>  21
<211>  20
<212>  DNA
<213>  Artificial Sequence

<220>
<223>  primer

<400>  21
cttgcatggc gtgttaaatg                                                 20

<210>  22
<211>  20
<212>  DNA
<213>  Artificial Sequence

<220>
<223>  primer

<400>  22
gcctccatga ggtcagatgt                                                   20


<210>  23
<211>  20
<212>  DNA
<213>  Artificial Sequence

<220>
<223>  primer

<400>  23
ggtgcacttg ggaaatttgt                                                   20


<210>  24
<211>  20
<212>  DNA
<213>  Artificial Sequence

<220>
<223>  primer

<400>  24
tatggacgaa tgcatggaaa                                                   20


<210>  25
<211>  20
<212>  DNA
<213>  Artificial Sequence

<220>
<223>  primer

<400>  25
tgacgaggac gatgagtacg                                                   20


<210>  26
<211>  20
<212>  DNA
<213>  Artificial Sequence

<220>
<223>  primer

<400>  26
gttgcgtctt gcatcatctg                                                   20


<210>  27

<211>  20
<212>  DNA
<213>  Artificial Sequence

<220>
<223>  primer

<400>  27
ctgtgggtga taaccgtgtg                                                   20


<210>  28
<211>  20
<212>  DNA
<213>  Artificial Sequence

<220>
<223>  primer

<400>  28
taaaagtagg gcccctgtcc                                                   20


<210>  29
<211>  20
<212>  DNA
<213>  Artificial Sequence

<220>
<223>  primer

<400>  29
aacgccatag ttgcttgctt                                                   20


<210>  30
<211>  20
<212>  DNA
<213>  Artificial Sequence

<220>
<223>  primer

<400>  30
acgccgtgat ggtagagaag                                                   20


<210>  31
<211>  18
<212>  DNA
<213>  Artificial Sequence

<220>
<223>  primer

<400>  31
catcctgggc tacactga                                                     18


<210>  32
<211>  18
<212>  DNA
<213>  Artificial Sequence

<220>
<223>  primer

<400>  32
ttgacaaagt ggtcgttg                                                    18


<210>  33
<211>  19
<212>  DNA
<213>  Artificial Sequence

<220>
<223>  primer

<400>  33
gtgccccatt aaccagttg                                                   19


<210>  34
<211>  20
<212>  DNA
<213>  Artificial Sequence

<220>
<223>  primer

<400>  34
ggcgtttaca gctcaagtcc                                                  20


<210>  35
<211>  18
<212>  DNA
<213>  Artificial Sequence

<220>
<223>  primer

<400>  35
cgctggacat gacttttg                                                    18


<210>  36
<211>  20
<212>  DNA
<213>  Artificial Sequence

<220>
<223>  primer

<400>  36
aggaacggtg tgcgcaggta                                                  20


<210>  37
<211>  20
<212>  DNA
<213>  Artificial Sequence

<220>
<223>  primer

<400>  37
cggctaccac atccaaggaa                                                    20

<210>  38
<211>  18
<212>  DNA
<213>  Artificial Sequence

<220>
<223>  primer

<400>  38
gctggaatta ccgcggct                                                      18